# EUROPÄISCHE PATENTSCHRIFT

(11) **EP 2 181 137 B1**
(45) Veröffentlichungstag und Bekanntmachung des Hinweises auf die Patenterteilung: **10.04.2013**
(21) Anmeldenummer: 08786808.9
(22) Anmeldetag: 04.08.2008
(51) Int. Cl.: A61K 8/86, A61Q 5/06, A61Q 5/12, A61Q 19/10, C11D 3/37, D06M 15/643, A61Q 5/00, C08G 18/48, C08G 18/71, C09D 175/04, C09D 5/16, C09D 175/08, C09D 201/02, C08G 18/10

(54) **MISCHUNGEN MULTIFUNKTIONELLER STERNFÖRMIGER PRÄPOLYMERE, DEREN HERSTELLUNG UND VERWENDUNG SOWIE BESCHICHTUNGEN DARAUS**
MIXTURES OF MULTIFUNCTIONAL STELLATE PREPOLYMERS PRODUCTION USE AND COATINGS MADE THEREOF
MÉLANGES DE POLYMÈRES ÉTOILÉS MULTIFONCTIONNELS, LEUR PRÉPARATION ET UTILISATION ET REVÊTEMENTS OBTENUS PAR CES MÉLANGES

(30) Priorität: 22.08.2007 DE 102007039648
(43) Veröffentlichungstag der Anmeldung: 05.05.2010
(73) Patentinhaber: Henkel AG & Co. KGaA, 40589 Düsseldorf (DE)
(72) Erfinder: RONG, Haitao, 64287 Darmstadt (DE); GREIWE, Peter, 69115 Heidelberg (DE); GROLL, Jürgen, 52074 Aachen (DE); MOHR, Christine, 36179 Bebra (DE); GLESIUS, Marina, 64291 Darmstadt (DE); MÖLLER, Martin, 52070 Aachen (DE)
(86) Internationale Anmeldenummer: PCT/EP2008/060194
(87) Internationale Veröffentlichungsnummer: WO 2009/024450

(56) Entgegenhaltungen:
- DE-A1-102004 031 938

## Beschreibung

Die vorliegende Erfindung betrifft Beschichtungen auf Basis von Mischungen unterschiedlicher, untereinander vernetzbarer sternförmiger Präpolymere und/oder sternförmiger Präpolymer-Nanoteilchen-Komplexe mit hydrophilen Polymerarmen, welche an ihren freien Enden hydrolysierbare Silyl- und/oder Siloxyl-Endgruppen tragen, sowie die Herstellung darauf basierender Beschichtungen. Des Weiteren betrifft die Erfindung die für derartige Beschichtungen geeigneten Mischungen sternförmiger Präpolymere, deren Herstellung und Verwendung in mannigfaltigen Anwendungsbereichen.

In diversen Anwendungsbereichen, wie beispielsweise der Medizin, der Bioanalytik, der Kosmetik, bei technischen Einrichtungen, der Textilausrüstung, bei Waschmitteln für Textilien, dem Haushaltsbereich, dem Hygienebereich und dem Gebiet Antifouling besteht der Bedarf, Oberflächen so auszurüsten, dass diese insbesondere Schmutz und mikrobielle Verunreinigungen, seien es Proteine oder Zellen, abweisen (Soil Repellency) bzw. deren Ablösung/Abwaschbarkeit zu erleichtern (Soil Release). Da gerade Schmutz, Proteine, diverse Polymere oder Zellen gewöhnlich gut auf hydrophoben Materialien haften, besteht ein besonderer Bedarf an hydrophil ausgestatteten Oberflächen.

Zu den bislang wirksamsten hydrophilen Beschichtungen gehören die auf Polyethylenoxiden beziehungsweise Polyethylenglykolen basierenden Hydrogel-Beschichtungen. Zur Herstellung derartiger Beschichtungen werden verschiedene Methoden vorgeschlagen.

WO 9952574 A1 beschreibt eine biomolekülabweisende Beschichtung, die durch Immobilisierung eines endständigen, mit Trichlorsilan modifizierten, linearen Polyethylenglykols auf glasartigen Oberflächen hergestellt wurde.

Aus der WO 9112886 A1 und WO 9325247 A1 ist eine Hydrogel-Beschichtung bekannt, die aus sternförmigen Polyethylenoxiden mit Hilfe einer Elektronenbestrahlung hergestellt wurde.

Die EP 335308 A2 beschreibt die Verwendung von Präpolymeren aus Polyethylenoxiddiolen und - triolen, deren terminale OH-Gruppen mit Polyisocyanaten umgesetzt wurden, für die Herstellung von Beschichtungen mit niedriger unspezifischer Proteinadsorption.

Die WO 03063926A1 offenbart eine ultradünne Hydrogel-Beschichtung, die aus sternförmigen isocyanat-terminierten Präpolymeren mit Polyether-Polymerarmen hergestellt wurde. Solche Hydrogel-Beschichtungen unterdrücken wirksam eine unspezifische Proteinabsorption auf damit ausgerüsteten Oberflächen.

Weiterhin wird in der DE 102004031938 A1 und der DE 10332849 A1 die Verwendung solcher Hydrogel-Beschichtung in Hygiene- und bioanalytischen Bereichen beschrieben.

Obwohl die aus dem Stand der Technik bekannten Hydrogel-Beschichtungen eine Verringerung der Zell- und Proteinadsorption in unterschiedlichem Maß bewirken, verhindern komplizierte Herstellungsverfahren für diese Beschichtungen in vielen Fällen eine breite Anwendbarkeit.

Hierzu zählt beispielsweise die Verwendung reaktiver, schlecht handhabbarer oder nur aufwendig synthetisierbarer Beschichtungsmaterialien, die Verwendung kostspieliger Bestrahlungsanlagen oder die zwingende Verwendung von Haftvermittlern, wodurch aufwendige Beschichtungsprozesse notwendig werden.

Eine haftvermittlerfreie Herstellung von hydrophilen Hydrogel-Beschichtungen, die auf Substratoberflächen stabil kovalent verankert sind, und die auf eine einfache Weise erhalten werden können, wodurch die Beschichtungsprozesse wesentlich vereinfacht werden und ein breites Anwendungsspektrum eröffnet wird, ist aus dem Stand der Technik nicht bekannt.

Es besteht daher auch ein Bedarf der Verbesserung des Herstellungsprozesses derartiger Hydrogel-Beschichtungen, wobei insbesondere auf den Einsatz von Haftvermittlern verzichtet werden kann und trotzdem langzeitstabile Beschichtungen erhalten werden.

Neben der verringerten Haftneigung von Mikroorganismen ist es aus reinigungstechnischen Gründen günstig, Oberflächen mit hydrophilen Eigenschaften zu versehen, da sich solche Oberflächen leicht mit den üblichen Waschflüssigkeiten auf wässriger Basis benetzen lassen und somit Abwaschprozesse erleichtern (Soil Release). Diese Oberflächen müssten jedoch gleichzeitig so ausgestattet sein, dass nach einer Benetzung das Wasser wieder möglichst vollständig ablaufen kann und somit kein Wasserfilm auf der Oberfläche verbleibt.

Die nach dem jetzigen Stand der Technik bekannten hydrophilen Oberflächen benetzen zwar mehr oder weniger vollständig mit Wasser beziehungsweise mit wasserbasierten Reinigungslaugen. Das Wasser bildet jedoch entweder einen stabilen Film auf der Oberfläche oder läuft nur in geringem Maße ab. Dies hat den Nachteil, dass beim Eintrocknen des Wasserfilms eine Rest-Anschmutzung auf der Oberfläche verbleibt. So bleiben unter anderem Mineralienablagerungen, wie beispielsweise Kalkablagerungen zurück, die eine erneute Anschmutzung - auch durch Proteine und Mikroorganismen - begünstigen. Aus diesem Grunde besteht ein Bedarf an hydrophilen Oberflächen, welche die Benetzung und Ablösung von Schmutz erleichtern, die aber gleichzeitig von einem Wasserfilm leicht "entnetzt" werden.

Aus Fabbri et al., J. Sol-Gel Science and Technologie 34 (2005) 155-163, ist eine leicht wasserentnetzende Beschichtung auf der Basis von Perfluorpolyethern und Silica (aus Tetraethoxyorthosilan, TEOS) bekannt, die jedoch einen großen Wasserkontaktwinkel, das heißt eine relativ hohe Hydrophobizität besitzt. Bei Fabbri et al. werden auch fluorfreie und reine TEOS-Schichten (also SiO_{2-x/2}(OH)ₓ) beschrieben, die bei Kontaktwinkeln von etwa 56-58° eine Hysterese von 3,6° besitzen.

Eine besondere Aufgabe der vorliegenden Erfindung bestand jedoch auch darin, den gewünschten Beschichtungen ein breites Eigenschaftsspektrum verleihen zu können, insbesondere die Benetzbarkeit, Wasserquellbarkeit, Protein- und Zellabweisungsfähigkeit sowie mechanische und thermische Stabilität gezielt einstellen zu können, um die Beschichtungen so auszurüsten, dass sie verschiedene Anforderungen gleichzeitig erfüllen können.

Die vorstehend genannte Aufgabe sowie die mit hohen Hydrophobizitäten und geringen Entnetzungseigenschaften verbundenen Nachteile des Stands der Technik werden in der vorliegenden Erfindung durch Bereitstellung von Beschichtungen überwunden, die eine mittels Sessile Drop-Methode und Tilting Plate Methode durch Verwendung eines Kontaktwinkelmessgerätes, ausgestattet mit einem kippbaren Messtisch, einem Videomeßsystem sowie automatischer Auswertungssoftware, gemessene Kontaktwinkelhysterese von Wasser von höchstens 20° besitzen und herstellbar sind aus einer Mischung umfassend mindestens zwei unterschiedlicher, untereinander und mit der Oberfläche des zu beschichtenden Substrates vernetzbarer sternförmiger Präpolymerer und/oder sternförmiger Präpolymer-Nanoteilchen-Komplexe, wobei die sternförmigen Präpolymere und/oder sternförmigen Präpolymer Nanoteilchen-Komplexe vor ihrer Vernetzung mindestens drei hydrophile Polymerarme besitzen, die für sich gesehen in Wasser löslich sind und die an allen oder einem Teil ihrer freien Enden Silyl-Endgruppen R¹ der folgenden allgemeinen Formel (I)

R¹ ist-CR^{a}₂-Si(OR^{b})ᵣ(R^{c})₃₋ᵣ (I)

tragen, wobei R^{a} für Wasserstoff oder eine lineare oder verzweigte Alkylgruppe mit 1 bis 6 Kohlenstoffatomen steht, OR^{b} für eine hydrolysierbare Gruppe steht, R^{c} für eine lineare oder verzweigte Alkylgruppe mit 1 bis 6 Kohlenstoffatomen steht und r für eine Zahl von 1 bis 3 steht, wobei die Silyl-Endgruppen R¹ nicht über ein Polyisocyanat, worunter hier und im Folgenden auch Diisocyanate zählen, am Ende des Polymerarms angebunden sind,
und an den gegebenenfalls vorhandenen nicht Silyl-Endgruppen-tragenden Enden reaktive bzw. funktionelle Gruppen tragen, welche gegenüber sich selbst, dem zu beschichtenden Substrat, optional in die Beschichtung eingebrachten Entities und/oder mit den Silyl-Endgruppen reaktiv sind, mit der Maßgabe, dass die Mischung: (a) mindestens ein sternförmiges Präpolymer, welches 3 bis 5 hydrophile Polymerarme besitzt und (b) mindestens ein sternförmiges Präpolymer und/oder einen sternförmigen Präpolymer-Nanoteilchen-Komplex mit mindestens 6 hydrophilen Polymerarmen, enthält.

Durch die Wahl zweier unterschiedlicher, wie oben definierter sternförmiger Komponenten (a) und (b) konnten hybridartige Beschichtungen erhalten werden, die gegenüber Beschichtungen aus nur einem einzelnen sternförmigen Präpolymer eine gezieltere Einstellung unterschiedlicher Eigenschaften erlauben und überraschenderweise eine verbesserte und damit auch stabilere Oberflächenbedeckung erlauben.

Sternförmige Präpolymere im Sinne dieser Erfindung sind solche, die Polymerarme an eine Zentraleinheit gebunden besitzen, wobei die Polymerarme im wesentlichen sternförmig beziehungsweise radial so an die Zentraleinheit gebunden sind, dass ein Ende des Polymerarms an die Zentraleinheit gebunden ist, während das andere Ende nicht an diese gebunden ist.

Sternförmige Präpolymer-Nanoteilchen-Komplexe im Sinne dieser Erfindung sind solche, die Polymerarme an ein Nanoteilchen gebunden besitzen, wobei die Polymerarme im wesentlichen sternförmig beziehungsweise radial so an das Nanoteilchen gebunden sind, dass ein Ende des Polymers an die Oberfläche des Nanoteilchens gebunden ist, während ein anderes Ende nicht an die Oberfläche des Nanoteilchens gebunden ist.

Bevorzugte Ausführungsformen erfindungsgemäßer Beschichtungen werden in den Ansprüchen 2 bis 22 sowie im Folgenden beschrieben.

Als bevorzugt in der Beschichtung einzusetzende sternförmige Präpolymere und/oder sternförmige Präpolymer-Nanoteilchen-Komplexe sind besonders solche geeignet, bei welchen das sternförmige Präpolymer und/oder der sternförmige Präpolymer-Nanoteilchen-Komplex mehrere an eine Zentraleinheit gebundene Polymerketten aufweisen und bei welchen im Falle des sternförmigen Präpolymers die Zentraleinheit vorzugsweise eine niedermolekulare organochemische Zentraleinheit und im Falle des sternförmigen Präpolymer-Nanoteilchen-Komplexes vorzugsweise ein anorganisches oxidisches Nanoteilchen ist.

Derartige bevorzugt in der erfindungsgemäßen Beschichtung einzusetzende sternförmige Präpolymere und/oder sternförmige Präpolymer-Nanoteilchen-Komplexe besitzen die nachfolgende allgemeine Formel (II):

(R²-B-A-X)ₙ-Z-(X-A-B-R¹)ₘ (II).

worin
Z für die Zentraleinheit steht, wobei diese im Fall der sternförmigen Präpolymere die Armanzahl der mehrarmigen Präpolymere festlegt,
A für einen hydrophilen, für sich gesehen in Wasser löslichen Polymerarm steht,
B und X unabhängig voneinander für eine chemische Bindung oder einen zweiwertigen, niedermolekularen organischen Rest mit vorzugsweise 1 bis 50 Kohlenstoffatomen steht,
wobei die Silyl-Endgruppen R¹ nicht über ein Polyisocyanat bzw. Diisocyanat am Ende des Polymerarms angebunden sind,
R² für eine mit R¹, dem Substrat und/oder mit sich selbst vernetzbare Gruppe steht,
und
m und n jewels ganze Zahlen sind, wobei im Fall der sternförmigen Präpolymere m ≥ 1 und n ≥ 0 und m+n einen Wert von 3 bis 100, im Falle, dass mindestens ein Rest R² für einen Isocyanat-Rest steht 4 bis 100 besitzt und mit der Armanzahl von Z übereinstimmt, und die m (X-A-B-R¹)-Gruppen sowie die n (X-A-B-R²)-Gruppen voneinander unabhängig verschiedene Bedeutung besitzen können, im Fall der Präpolymer-Nanoteilchen-Komplexe m ≥ 1 und n ≥ 0 und m+n einen Wert von 3 bis zu einem Maximalwert von 500 000 hat.

Im Falle der sternförmigen Präpolymere steht Z vorzugsweise für einen Glycerol-Rest, einen mehrwertigen Zucker, wie beispielsweise Sorbitol oder Sucrose. Prinzipiell können jedoch alle aus der Literatur zu Herstellung von sternförmigen Präpolymeren eingesetzten Starter-Moleküle eingesetzt werden, um den Rest Z zu bilden.

Im Falle der sternförmigen Präpolymer-Nanoteilchen-Komplexe steht Z vorzugsweise für ein Silica-, Zinkoxid-, Aluminiumoxid-, Zirkonoxid-, Calciumcarbonat-, Titandioxid-, Kohlenstoff-, Magnesiumoxid-, oder Eisenoxid-Nanoteilchen. Die Nanoteilchen der Gruppe Z sind entweder kommerziell erhältlich oder werden in-situ oder ex-situ vorzugsweise durch Sol-Gel-Verfahren, Fällung aus wässriger und nicht wässriger Lösung, Gasphasensynthese (Flammenpyrolyse, Chemical Vapour Deposition, etc.), mechanische Bearbeitung (z.B. Mahlen, Ultraschall) hergestellt. Besonders bevorzugt besitzen diese eine Größe von 0,5 bis 200 nm, ganz besonders bevorzugt von 0,5 bis 20 nm.

Im Falle der sternförmigen Präpolymer-Nanoteilchen-Komplexe werden die Polymerarme A vorzugsweise über hydrolysierbare Silylendgruppen an die Nanoteilchen-Oberfläche des Rests Z angebunden. Eine Anbindung kann jedoch auch über andere mit der Oberfläche reaktive Gruppen wie beispielsweise Carboxylgruppen, kationische Gruppen (z.B. Trialkylammoniumgruppen), Phosphonatgruppen, etc. erfolgen. Besonders zur Einführung der Polymerarme am Nanopartikel geeignet sind lineare Polyoxyalkylendiole, deren beide OH-Gruppen mit gegenüber OH-Gruppen reaktiven Silanen, wie beispielsweise Isocyanatosilanen umgesetzt sind. Andere zur Einführung der Polymerarme am Nanopartikel geeignete Verbindungen umfassen Polyether-Polyol beispielsweise VORANOL®, TERRALOX®, SYNALOX® und DOWFAX® der Dow-Chemical Corporation, SORBETH® der Glyco-Chemicals Inc., GLUCAM® der Amerchol Corp., Lupranol® und Pluronic® der BASF.

Die Benetzbarkeit der erfindungsgemäßen Beschichtungen mit Wasser ist ein empfindliches Maß für deren Hydrophilie oder Hydrophobie. Der Kontaktwinkel eines Wassertropfens auf einem planaren Substrat im umgebenden Medium Luft resultiert aus den Oberflächenenergien der Beschichtung und des Wassers sowie der Grenzflächenenergie zwischen Wasser und der Beschichtung nach der Youngschen Gleichung. Im Fall maximaler Hydrophilie geht der Kontaktwinkel gegen 0°. Im Fall maximaler Hydrophobie geht der Kontaktwinkel gegen 180°. In der vorliegenden Erfindung lässt sich die Hydrophilie bzw. Hydrophobizität der Beschichtungen auch durch Wahl unterschiedlicher sternförmiger Präpolymere beeinflussen. So sind die sternförmigen Präpolymere, welches 3 bis 5 hydrophile Polymerarme besitzen - bei gleichem Polymerarmaufbau und gleicher Polymerarmlänge - in der Regel hydrophober, als die sternförmigen Präpolymere (b) mit mindestens 6 hydrophilen Polymerarmen. Dieser Umstand lässt sich bei der Beschichtung unterschiedlicher Oberflächen ausnutzen, indem je nach zu beschichtender Oberfläche das Verhältnis der Mischungskomponenten (a) und (b) variiert wird.

In der Praxis wird häufig das Sessile-Drop-Verfahren bzw. das Tilting-Plate-Verfahren verwendet, um den statischen Kontaktwinkel und die Kontaktwinkelhysterese eines Flüssigkeitstropfens auf einer Oberfläche zu ermitteln. Wird z. B. ein Wassertropfen auf eine waggerecht liegende reale Oberfläche abgesetzt, bildet das Wasser einen symmetrischen Tropfen und der gemessene Kontaktwinkel wird als statischer Kontaktwinkel bezeichnet (Sessile Drop Methode). Während sich die Oberfläche langsam neigt, verformt sich der Wassertropfen dementsprechend asymmetrisch. Ab einem bestimmten Kippwinkel fängt der Tropfen an, sich in Bewegung zu setzen. Man misst zwei Kontaktwinkel, einen größeren (den fortschreitenden (advancing)) und einen kleineren (den rückziehenden (receding)). Im Idealfall sollte der Unterschied zwischen beiden gleich Null sein. Im Realfall existiert ein Unterschied, auch Kontaktwinkelhysterese genannt, der auf Oberflächenrauigkeit, Inhomogenitäten und Verunreinigungen zurückgeführt wird. Je kleiner der Wert der Hysterese ist, desto besser "entnetzt" sich die Beschichtung von anhaftendem Wasser.

Vorzugsweise besitzen die erfindungsgemäßen Beschichtungen einen statischen Wasserkontaktwinkel von höchstens 90°, besser höchstens 70°, besonders bevorzugt höchstens 50° und ganz besonders bevorzugt von höchstens 45°. Es werden in vielen Fällen jedoch auch Wasserkontaktwinkel von 40° und weniger erreicht.

Bevorzugt sind erfindungsgemäße Beschichtungen deren nach Sessile Drop Methode und Tilting Plate Methode gemessene Kontaktwinkelhysterese in Wasser höchstens 20°, besonders bevorzugt höchstens 18° und ganz besonders bevorzugt höchstens 15° beträgt. Es werden in weiter bevorzugten Fällen jedoch auch Kontaktwinkelhysteresen von höchstens 10° oder auch von 2°, 3° oder 4° und weniger erreicht.

In einer besonderen Ausführungsform werden die Beschichtungen, aus Mischungen sternförmiger Präpolymere der Anspruchs 1 oder der allgemeinen Formel (II) erhalten, wobei der Rest OR^{b} ein Alkoxy-Rest, besonders bevorzugt ein Methoxy- oder Ethoxy-Rest ist und r = 1, 2 oder 3, besonders bevorzugt 2 oder 3 ist. Beispiele für Reste R¹ sind Dimethylethoxysilyl-CR^{a}₂-, Dimethylmethoxysilyl-CR^{a}₂-, Diisopropylethoxysilyl-CR^{a}₂-, Methyldimethoxysilyl-CR⁹₂-, Methyldiethoxysilyl-CR^{a}₂-, Trimethoxysilyl)-CR^{a}₂-, Triethoxysilyl-CR^{a}₂- oder Tri-t-Butoxysilyl-CR^{a}₂-Reste.

B steht im sternförmigen Präpolymer der allgemeinen Formel (II) für eine chemische Bindung oder einen zweiwertigen, niedermolekularen organischen Rest mit vorzugsweise 1 bis 50, insbesondere 2 bis 20 C-Atomen. Beispiele zweiwertiger niedermolekularer organischer Reste umfassen aliphatische, heteroaliphatische, araliphatische, heteroaraliphatische, cycloaliphatische, cycloheteroaliphatische sowie aromatische und heteroaromatische Reste. Besonders bevorzugt sind kurzkettige aliphatische und heteroaliphatische Reste. Beispiele für geeignete Reste umfassen -Aminopropyl-, -N-(2-Aminoethyl)(3-aminopropyl)-, -3-Methacryloxypropyl-, -Methacryloxymethyl-, -3-Acryloxypropyl-, -3-Isocyanatopropyl-, -Isocyanatomethyl-, -Butyraldehyde-, -3-Glycidoxypropyl-, -Propylbernsteinsäureanhydrid-, -Chloromethyl-, -3-Chloropropyl-, -Hydroxymethyl-.

Insbesondere sind solche Beschichtungen bevorzugt, die aus Mischungen sternförmiger Präpolymere und/oder sternförmiger Präpolymer-Nanoteilchen-Komplexe der allgemeinen Formel (II) erhalten werden, bei welchen zwei benachbarte oder alle Reste B in der Gruppe B-R¹ nicht mehr als eine, vorzugsweise keine Wasserstoffbrücken zueinander aufbauen können. Eine solche Beschichtung mit einer geringen Quervernetzung durch Wasserstoffbrücken ermöglicht eine höhere Flexibilität in der Orientierung der Polymerarme A, was wiederum eine gleichmäßigere Verteilung der Präpolymere beziehungsweise Präpolymer-Nanoteilchen-Komplexe und den Erhalt einer gleichmäßigen, geschlossenen Beschichtung zur Folge hat. Des Weiteren kann das Vorliegen einer besonders großen Anzahl an Quervernetzungen beziehungsweise besonders starker Quervernetzungen durch Wasserstoffbrückenbindungen dazu führen, dass die Materialien zu viskos werden, um in typischen Anwendungsformulierungen Verwendung finden zu können.

Besonders bevorzugt sind daher solche Beschichtungen, in welchen der Rest B der sternförmigen Präpolymere der allgemeinen Formel (II) in der Gruppe B-R¹ höchstens eine Urethan-, eine Ester oder eine Harnstoffgruppe enthalten.

In einer weiteren bevorzugten Ausführungsform betrifft die vorliegende Erfindung Beschichtungen aus vernetzten sternförmigen Präpolymeren der allgemeinen Formel (II), in welchen der Rest R² vorzugsweise gewählt ist aus der Gruppe bestehend aus Isocyanat-Resten, (Meth)acrylat-Resten, Oxiran-Resten, alkoholischen OH-Gruppen, primären und sekundären Aminogruppen, Thiolgruppen und Silangruppen. Werden als R²-Gruppen Silangruppen eingesetzt, so können diese auch die allgemeine Formel (I) besitzen, müssen sich jedoch von R¹ in wenigstens einer der Gruppen R^{a}, R^{b} und R^{c} und/oder dem Zahlenwert von r unterscheiden. Als weitere Gruppen R² sind beispielsweise geeignet Oxazolingruppen, Carbonsäuregruppen, Carbonsäureester-, Lactone- , Lactame-, Carbonsäureanhydridgruppen, Carbonsäure- und Sulfonsäurehalogenid-Gruppen Aktivestergruppen, radikalisch polymerisierbare C=C-Doppelbindungen, z. B. neben den oben genannten (Meth)acrylgruppen auch Vinylether- und Vinylestergruppen, weiterhin aktivierte C=C-Doppelbindungen, aktivierte C≡C-Dreifachbindung und N=N-Doppelbindungen, die mit Allylgruppen im Sinne einer en-Reaktion oder mit konjugierten Diolefin-Gruppen im Sinne einer Diels-Alder-Reaktion reagieren. Beispiele für Gruppen, die mit Allylgruppen im Sinne einer en-Reaktion oder mit Dienen im Sinne einer Diels-Alder-Reaktion reagieren können, sind Maleinsäure- und Fumarsäure-Gruppen, Maleinsäureester- und Fumarsäureester-Gruppen, Zimtsäureestergruppen, Propiolsäure(ester)gruppen, Maleinsäureamid- und Fumarsäureamid-Gruppen, Maleinimid-Gruppen, Azodicarbonsäureester-Gruppen und 1,3,4-Triazolin-2,5-dion-Gruppen. Besonders bevorzugt ist R² in Beschichtungen eine Isocyanat-, Oxiran- oder OH-Gruppe.

Ein Vorteil der erfindungsgemäßen Hydrogel-Beschichtung gegenüber bekannten Hydrogel-Beschichtungen liegt darin, dass deren Eigenschaften gezielt durch eine entsprechende Auswahl der R'- und R²-Reste sowie deren Verhältnis zueinander sowie das Verhältnis der Mischungskomponenten (a) und (b) definierbar sind. So kann beispielsweise die Benetzbarkeit, Wasserquellbarkeit und die Protein- und Zellabweisungsfähigkeit durch gezielte Einstellung des R¹/R²-Verhältnisses beeinflusst werden.

Die erfindungsgemäßen Beschichtungen enthalten Mischungen sternförmiger Präpolymere, deren Polymerarme, für sich gesehen, in Wasser löslich sind. Die bevorzugten sternförmigen Präpolymere der allgemeinen Formel (II) besitzen vorzugsweise Polymerarme A, die ausgewählt sind aus der Gruppe bestehend aus Poly-C₂-C₄-alkylenoxiden, Polyoxazolidonen, Polyvinylalkoholen, Homo- und Copolymeren, die wenigstens 50 Gew.-% N-Vinylpyrrolidon einpolymerisiert enthalten, Homo- und Copolymeren, die wenigstens 30 Gew.-% Acrylamid und/oder Methacrylamid einpolymerisiert enthalten, Homo- und Copolymeren, die wenigstens 30 Gew.-% Acrylsäure und/oder Methacrylsäure einpolymerisiert enthalten. Hierbei sind besonders bevorzugt Polymerarme A die aus Polyethylenoxid oder Ethylenoxid/Propylenoxid-Copolymeren bestehen. Werden die ganz besonders bevorzugten Ethylenoxid/Propylenoxid-Copolymeren eingesetzt, so empfiehlt sich ein Propylenoxidanteil von höchstens 50 Gew.-% vorzugsweise höchstens 40 Gew.- % und besonders bevorzugt höchstens 25 Gew.-%, bezogen auf die Summe des Gewichts aus Propylenoxideinheiten (iso-C₃H₆O) und Ethylenoxideinheiten (C₂H₄O).

Die Indices m und n der in den Beschichtungen eingesetzten sternförmigen Präpolymere und/oder sternförmigen Präpolymer-Nanoteilchen-Komplexe stehen jeweils für ganze Zahlen, wobei m ≥ 1 und n ≥ 0, und m+n im Fall der sternförmigen Präpolymere vorzugsweise einen Wert von 3 bis 100 und im Fall der Präpolymer-Nanoteilchen-Komplexe vorzugsweise einen Wert von 3 bis zu einem Maximalwert von 500 000. besitzt.

Im Falle der sternförmigen Präpolymere stehen die Indices m und n jeweils für ganze Zahlen, wobei m ≥ 1 und n ≥ 0, und m+n vorzugsweise einen Wert von 3 bis 100, oder 3 bis 50, insbesondere 3 bis 10 besitzt und mit der Armanzahl von Z übereinstimmt. Die Zentraleinheit besitzt daher in der Regel 3 bis 100, vorzugsweise 3 bis 50, insbesondere 3 bis 10 Gerüstatome die als Anknüpfungspunkte für die Arme dienen. Der in der erfindungsgemäßen Beschichtung oder der erfindungsgemäßen Mischung eingesetzte Mischungsbestandteil (a) - das sternförmige Präpolymer mit 3 bis 5 hydrophilen Polymerarmen - besitzt vorzugsweise 3 oder 4, besonders bevorzugt 3 hydrophile Polymerarme. Der weitere Mischungsbestandteil (b) - das sternförmige Präpolymer und/oder der sternförmige Präpolymer-Nanoteilchen-Komplex mit mindestens 6 hydrophilen Polymerarmen - besitzt vorzugsweise 6 bis 10, besonders bevorzugt 6 bis 8 Polymerarme.

Im Falle der sternförmigen Präpolymer-Nanoteilchen-Komplexe stehen die Indices m und n jeweils für ganze Zahlen, wobei m ≥ 1 und n ≥ 0, und m+n vorzugsweise einen Wert von 3 bis 500 000 besitzen.

In einer besonderen Ausführungsform ist n gleich 0, wobei das sternförmige Präpolymer einem komplett R'-modifizierten Präpolymer entspricht, das vorzugsweise 5 bis 50 und besonders bevorzugt 4 bis 10 Polymerarme aufweist. Im Fall n>0, bewegt sich das Verhältnis n/m zwischen 99/1 und 1/99, vorzugsweise 49/1 und 1/49, und insbesondere 9/1 und 1/9.

Die sternförmigen Präpolymere der erfindungsgemäßen Beschichtungen und der erfindungsgemäßen Mischungen lassen sich beispielsweise durch Umsetzung von Verbindungen der allgemeinen Formel Z-(X-A-Y)ₘ₊ₙ, worin Z, X, A, m und n wie oben definiert sind, mit gegenüber der Gruppe Y reaktiven Silanen erhalten. Die Gruppe Y ist dabei vorzugsweise eine Hydroxy- oder Aminogruppe. Als reaktives Silan ist in einem solchen Fall beispielsweise Isocyanato-Silane wie (3-Isocyanatopropyl)tiralkyoxysilan, darunter (3-Isocyanatopropyl)trimethoxysilan, (3-Isocyanatopropyl)triethoxysilan, (Isocyanatomethyl)methyl-Dimethoxysilan und (Isocyanato-methyl)trimethoxysilan, Aldehyde-Silane wie Triethoxysilylundecanal und Triethoxysilylbutyraldehyde, Epoxy-Silane wie (3-Glycidoxypropyl)trimethoxysilan, Anhydridsilane wie 3-(Triethoxysilyl)propylbernsteinsäureanhydrid, Halogen-Silane wie Chloromethyltrimethoxysilan, 3-Chloropropylmethyldimethoxysilan, geeignet. Wenn die Gruppe Y für OH steht, so handelt es sich bei den Verbindungen der allgemeinen Formel Z-(X-A-Y)ₘ₊ₙ vorzugsweise um Polyether-Polyole. Die Verbindugnen der allgemeinen Formel Z-(X-A-Y)ₘ₊ₙ besitzen vorzugsweise ein zahlenmittleres Molekulargewicht im Bereich von 200 bis 50000, besonders bevorzugt 1000 bis 30000 und ganz besonders bevorzugt 5000 bis 20000 g/mol aufweist. Das zahlenmittlere Molekulargewicht wird dabei wie im Beispielteil angegeben bestimmt.

Das sternförmige Präpolymer enthält vorzugsweise wenigstens 0,05 Gew.-%, besonders bevorzugt wenigstens 0,1 Gew.-% und ganz besonders bevorzugt wenigstens 0,15 Gew.-% Silizium.

In einer besonderen Ausführungsform enthält die erfindungsgemäße Beschichtung zusätzlich Fremdmaterialien organischen, anorganischen oder natürlichen Ursprungs, die im Folgenden schlicht als Entities bezeichnet werden. Ein Entity ist vorzugsweise gewählt aus der Gruppe bestehend aus biologisch aktiven Substanzen, Pigmenten, Farbstoffen, Füllstoffen, Kieselsäureeinheiten, Nanopartikeln, Organosilanen, biologischen Zellen, Rezeptoren oder Rezeptor tragenden Molekülen oder Zellen und physikalisch in die Beschichtung eingelagert und/oder an diese oder in dieser kovalent gebunden.

Beispiele für derartige Entities sind bioaktive Materialien wie Wirkstoffe, Biozide, Oligonukleotide, Peptide, Proteine, Signalstoffe, Wachstumsfaktoren, Zellen, Kohlenhydrate und Lipide, anorganische Komponenten wie Apatite und Hydroxylapatite, quartäre Ammoniumsalzverbindungen, Verbindungen aus Bisguanidinen, quartäre Pyridiniumsalzverbindungen, Verbindungen aus Phosphoniumsalzen, Thiazoylbenzimidazole, Sulfonylverbindungen, Salicylverbindungen oder metallorganische und metallanorganische Verbindungen. Bevorzugt sind antibakteriellwirkende Stoffe wie beispielsweise Peptide, Metallkolloide und quartäre Ammonium- und Pyridinumsalzverbindungen.

Eine weitere wesentliche Gruppe von Entities stellen organisch funktionalisierte Silane (Organosilane) vom Typ (R')₁₊ₓSi(OR")₃₋ₓ (x = 0, 1 oder 2) dar. Kennzeichnend hierbei ist das gleichzeitige Vorliegen von Kieselsäureestergruppen (OR"), die in wässriger Lösung zu kondensationsfähigen Silanolgruppen (Si-OH) hydrolysieren, sowie von hydrolysestabilen Si-R'-Bindungen am gleichen Siliziumatom, wobei letztere hydrolysestabile Bindung in der Regel in einer kovalenten Si-C-Einfachbindung besteht. Häufig stellen die genannten funktionalisierten Silane niedermolekulare Verbindungen dar, jedoch fallen auch oligomere oder polymere Verbindungen unter den Begriff "organisch funktionalisierte Silane", wesentlich ist, dass im selben Molekül sowohl zu Silanolgruppen hydrolysierbare Si-OR"-Gruppen sowie nicht hydrolysierbare Si-R'-Gruppen vorliegen. Durch die in der Regel organischen R'-Gruppen der funktionalisierten Silane gelingt es, die ganze Bandbreite zusätzlicher chemischer Funktionalitäten in die hier beschriebenen Beschichtungen einzubauen. Beispielsweise können kationische Haftgruppen (beispielsweise - NR'''₃+-Gruppen), anionische Haftgruppen (beispielsweise -SO₃⁻), redoxaktive Gruppen (z.B. Chinon/Hydrochinonreste), Farbstoffgruppen (beispielsweise Azofarbstoffmoleküle. Aufheller auf Stilbenbasis), Gruppen mit biologischer/pharmakologischer Wirksamkeit (beispielsweise auch Saccharid- bzw. Polysaccharidmoleküleinheiten, Peptide bzw. Proteineinheiten und andere organische Strukturmotive), Gruppen zur kovalenten Anbindung an Substrate (beispielsweise Epichlorhydrinreste, Cyanurchlorid Cystin/Cysteineinheiten und dergleichen), Gruppen mit bakterizider Wirksamkeit (beispielsweise NR'''₃⁺-Gruppen mit sehr langen R'''-Alkylresten), katalytisch wirksame Gruppen (beispielsweise Übergangsmetallkomplexe mit organischen Liganden) auf diese Art in die Schicht eingebaut werden. Weitere über den Rest R' eingeführte Gruppen umfassen beispielsweise Epoxy-, Aldehyd-, Acrylat- und Methacrylat-Gruppen, Anhydrid-, Carboxylat- oder Hydroxy-Gruppen. Die hier beschriebenen Funktionalitäten sind im Sinne einer Auswahl von Beispielen zu verstehen keinesfalls als vollständige Auflistung. Die Organosilane dienen daher nicht nur als Vernetzungshilfe, sondern gleichzeitig als Funktionalitätsverleiher. Man erhält auf diese Weise direkt eine erfindungsgemäße Hydrogelbeschichtung mit gewünschten Funktionalitäten.

Zu den Entities gehören ebenfalls nanopartikuläre Metall- oder Halbmetalloxide. Beispielsweise sind diejenigen von Silizium, Zink, Titan, Aluminium, Zirkonium geeignet. Insbesondere Siliziumoxidpartikel mit einem Durchmesser von etwa 1 bis 500 nm sind bevorzugt. Solche SiO₂-Partikel, einschließlich deren oberflächenmodifizierte bzw. -funktionalisierte Derivate, können zur Verbesserung der mechanischen Eigenschaften der Schichten beitragen.

Eine weitere Gruppe von Entities stellen anorganische Pigmente dar. Die erfindungsgemäßen Beschichtungen mit reaktiven Silylgruppen binden leicht über stabile kovalente Bindungen an diese an. Bringt man ein erfindungsgemäßes Hydrogel, das heißt eine erfindungsgemäße Beschichtung, welche mit Pigmenten vermischt, ist auf eine Oberfläche auf, auf der das Hydrogel anbinden kann, so erhält man auf diese Weise gebundene, pigmentierte Oberflächenbeschichtungen. Falls organische Pigmente in das Hydrogel eingebaut werden sollen, beziehungsweise falls eine Haftung des Hydrogels auf organischen Oberflächen gewährleistet werden soll, so können in die erfindungsgemäße Beschichtung Organosilane mit entsprechenden Haftgruppen eingebunden werden (z.B. kationischen Gruppen, wie oben beschrieben). Auf diese Weise werden Mittel und Verfahren möglich, durch die sich Pigmente beispielsweise auf Haaren gut verankern lassen. Bindet man beispielsweise Glimmer oder Effektpigmente (Perlglanzpigment) auf Haar an, so werden besondere optische Effekte auf Haar ermöglicht ("Glitzerhaar") Durch die Verwendung farbiger anorganischer oder organischer Pigmente (beispielsweise Lapis Lazuli, Pyrolopyrrole) werden besonders intensive, beziehungsweise stabile Haarfarben erhalten.

Der Einbau der Entities erfolgt vorzugsweise durch Co-Adsorption aus Lösungen, die Mischungen der sternförmigen Präpolymere und/oder der sternförmigen Präpolymer-Nanopartikel-Komplexe und den Fremdbestandteil enthalten. Außerdem können die sternförmigen Präpolymere und/oder Präpolymer-Nanopartikel-Komplexe mit den genannten bioaktiven Materialien chemisch umgesetzt werden oder als Mischung mit nicht modifizierten sternförmigen Präpolymeren und/oder Präpolymer-Nanopartikel-Komplexen auf der Oberfläche zur Reaktion gebracht werden. Selbstverständlich ist es auch möglich, die Fremdstoffe gezielt durch Physisorption oder Chemisorption auf die fertige erfindungsgemäße Hydrogel-Beschichtung aufzubringen.

Die mit den erfindungsgemäßen Beschichtungen zu beschichtenden Substrate unterliegen grundsätzlich keinen Einschränkungen. Die Substrate können regelmäßig oder unregelmäßig geformte, glatte oder poröse Oberflächen aufweisen.

Geeignete Oberflächenmaterialien sind beispielsweise glasartige Oberflächen, wie Glas, Quarz, Silicium, Siliciumdioxid oder Keramik, oder Halbleitermaterialien, Metalloxide, Metalle und Metalllegierungen wie Aluminium, Titan, Zirkonium, Kupfer, Zinn und Stahl. Auch Verbundwerkstoffe wie glasfaserverstärkte oder carbonfaserverstärkte Kunststoffe (GFK, CFK), Polymere wie Polyvinylchlorid, Polyethylen, Polymethylpentene, Polypropylen, allgemein Polyolefine, elastomere Kunststoffe wie Polydimethylsiloxan, Polyester, Fluorpolymere, Polyamide, Polyurethane, Poly(meth)acrylate sowie Copolymere, Blends und Komposite der vorgenannten Materialien eignen sich als Substrate. Darüber hinaus können Zellulose und natürliche Fasern wie Baumwollfasern, Wolle und Haare als Substrate eingesetzt werden. Aber auch mineralische Oberflächen, wie Anstriche oder Fugenmaterial können als Substrate dienen. Für Polymersubstrate ist es in einigen Fällen ratsam, die Oberflächen vorzubehandeln. Besonders bevorzugte Substratmaterialien sind glasartige beziehungsweise generell anorganische Oberflächen, da bei diesen unmittelbar eine Anbindung über relativ hydrolysestabile Bindungen beispielsweise Si-O-Si, oder Si-O-Al erfolgt und somit eine Oberflächenvorbehandlung nicht nötig ist. Falls nicht wie oben beschrieben eine direkte Ausbildung (hydrolysestabiler) kovalenter Bindungen zwischen Hydrogel und Substrat gelingt, also beispielsweise beim Vorliegen organischer Substratoberflächen (Si-O-C-Bindungen sind hydrolyselabil), so kann die Anbindung in vorteilhafter Weise durch Zugabe organofunktioneller Silane, die über Haftgruppen verfügen, erfolgen. Geeignete Haftgruppen sind beispielsweise kationische Trimethylammoniumgruppen oder Aminogruppen. Durch das gleichzeitige Vorliegen von reaktiven Siloxylgruppen werden diese funktionellen Gruppen in das Hydrogel eingebaut und werden gleichsam integraler, kovalent gebundener Bestandteil der Beschichtung.

Insbesondere im Bereich der Glas-, Keramik-, Kunststoff- und Metallsubstrate bietet sich beispielsweise eine Anwendung in der Ausstattung von Duschen, Fenstern, Aquarien, Gläsern, Geschirr, Waschbecken, Toiletten, Arbeitsoberflächen, oder Küchengeräten, wie beispielsweise Kühlschränken oder Herden mit einer leicht reinigbaren temporären oder permanenten Ausstattung an, die ein vollständiges Ablaufen von Wasser ermöglicht, sowie Proteine und Bakterien abweist.

Weiterer Gegenstand der vorliegenden Erfindung ist ein Verfahren zur Herstellung der erfindungsgemäßen Beschichtungen auf einem Substrat, wobei eine Lösung der Mischung aus (a) und (b) (wie oben definiert) auf das zu beschichtende Substrat aufgebracht wird, und vorher, gleichzeitig oder anschließend eine zumindest teilweise Vernetzungsreaktion der Silyl-Endgruppen und der gegebenenfalls vorhandenen reaktiven Gruppen der nicht Silyl-Endgruppen-tragenden Enden untereinander und/oder mit dem Substrat erfolgt.

Bevorzugte Ausführungsformen des erfindungsgemäßen Verfahrens sind in Ansprüchen 24 bis 32 sowie im Folgenden beschrieben.

Bevorzugt wird das Verfahren mit den Mischungen der sternförmigen Präpolymere und/oder sternförmigen Präpolymer-Nanoteilchen-Komplexe der allgemeinen Formel (II) durchgeführt.

In einer bevorzugten Ausführungsform des erfindungsgemäßen Verfahrens werden vor, während und/oder nach dem Aufbringen der Lösung der Mischung aus (a) und (b) auf das zu beschichtende Substrat ein Fremdmaterial, beispielsweise ein Entity gewählt aus der Gruppe bestehend aus biologisch aktiven Substanzen, Pigmenten, Farbstoffen, Füllstoffen, Kieselsäureeinheiten, Nanopartikeln, Organosilanen, biologischen Zellen, Rezeptoren oder Rezeptor tragenden Molekülen oder Zellen, oder Vorstufen des vorgenannten Entities mit den sternförmigen Präpolymeren in Kontakt gebracht. Die eingebrachten Entities können hierbei physikalisch in das Netzwerk der vernetzten sternförmigen Präpolymeren und/oder sternförmigen Präpolymer-Nanoteilchen-Komplexe eingelagert sein oder an die Oberfläche der Beschichtung ionisch durch van der Waals- oder Wasserstoffbrückenbindungen gebunden sein, oder aber chemisch über kovalente Bindungen, vorzugsweise über reaktive Endgruppen des sternförmigen Präpolymeren gebunden werden.

Werden beispielsweise Kieselsäureeinheiten als Entities in die Beschichtung eingebracht, so kann dies durch Vermischen einer Lösung der Mischung aus (a) und (b) mit einem hydrolysierbaren Kieselsäurevorläufer, wie beispielsweise einem Tetraalkoxysilan (zum Beispiel Tetraethoxyorthosilan; TEOS), vorzugsweise in Gegenwart eines Katalysators, wie beispielsweise einer Säure, oder einer Base, erfolgen. Das Gewichtverhältnis an SiO₂ der eingebrachten Kieselsäureeinheiten bezogen auf den Polyethylen/Polypropylenoxid-Anteil in der Beschichtung beträgt vorzugsweise 0,01 bis 100, besonders bevorzugt 0,5 bis 50, und ganz besonders bevorzugt 1 bis 10. Die Anbindung der Kieselsäureeinheiten an das sternförmige Präpolymer kann dabei über van der Waals-Bindungen, ionisch oder über Wasserstoffbrücken erfolgen. Vorzugsweise erfolgt die Bindung jedoch kovalent über eine -CSi-O-Si-Konstellation (Nachweis Raman oder IR) an reaktive Endgruppen der in den erfindungsgemäßen Beschichtungen eingesetzten sternförmigen Präpolymere und/oder sternförmigen Präpolymer-Nanoteilchen-Komplexe.

Der Wasserkontaktwinkel einer erfindungsgemäßen Beschichtung beträgt mittels des Sessile-Drop-Verfahrens gemessen an einer planaren, glatten Oberfläche gemessen vorzugsweise 0,0001 bis 90°, besonders bevorzugt 0,001 bis 70° und ganz besonders bevorzugt bis 50° oder nicht mehr als 45°. Die Wasserkontaktwinkelhysterese, gemessen nach dem Tilting-Plate-Verfahren, beträgt vorzugsweise nicht mehr als 18°, besonders bevorzugt nicht mehr als 15°.

Die Bindung der Kieselsäureeinheiten untereinander kann in der Beschichtung über Wasserstoffbrücken oder über ionische Wechselwirkung erfolgen. Allerdings sind kovalente -Si-0-Si-Brücken bevorzugt (nachweisbar durch IR). Die Wirkung des TEOS innerhalb der Schicht kann als Vernetzerwirkung verstanden werden, wobei Schichten ohne Vernetzer (TEOS) gewöhnlich hydrophiler sind, das heißt sich durch einen niedrigeren Kontaktwinkel auszeichnen. Generell lässt sich sagen, dass der Einbau zusätzlicher Vernetzer beispielsweise TEOS beziehungsweise funktioneller Alkoxysilane eine weitere Möglichkeit darstellt, die Eigenschaften der Beschichtungen individuell einzustellen.

Das Aufbringen der ultradünnen Hydrogel-Beschichtungen auf das Substrat erfolgt beispielsweise durch Abscheiden der Mischungen aus (a) und (b) nach an sich bekannten Verfahren auf der zu beschichtenden Oberfläche aus einer Lösung der Präpolymere, die darin schon zum Teil vorvernetzt sein können, und gleichzeitigem oder anschließendem Vernetzen der reaktiven Gruppen untereinander und mit der Substratoberfläche.

Generell können alle bekannten Beschichtungsverfahren eingesetzt werden. Beispiele hierfür sind Tauchbeschichtung, Spincoating, Einpolieren und Sprühverfahren. Zur Erzielung der gewünschten Eigenschaften der Oberflächenschicht wird man die Beschichtungsmaßnahmen so wählen, dass die Beschichtungsdicke, vorzugsweise einen Wert von 500 µm, besonders bevorzugt 200 µm und ganz besonders 100 µm nicht überschreitet. Je nach Anwendungszwecken muss eine Beschichtung gleichzeitig viele unterschiedliche Anforderungen hinsichtlich beispielsweise der mechanischen Eigenschaften, des Wasserbenetzung- und Wasserentnetzungsverhaltens, der Protein- und Bakterienabweisung und dergleichen erfüllen. Für viele Fälle, insbesondere im Haushaltsbereich, ist eine ultradünne oder dünne Schicht mit einer Schichtdicke von 0,1 bis 100 nm, insbesondere von 1 bis 50 nm oftmals ausreichend, um die gewünschten Effekte zu erzielen, während bei Anwendungen, beispielsweise aufgrund einer hohen mechanischen Beanspruchung der Oberfläche, dickere Schichten mit einer Schichtdicke beispielsweise von 50-500 µm erwünscht sind, wobei für manche Anwendungen, beispielsweise solche, die eine Anwesenheit von Nanopartikeln in der Beschichtung vorsehen auch größere Schichtdicken wie beispielsweise 1000 µm erwünscht sein können. Im Gegensatz zu anderen aus dem Stand der Technik bekannten hydrophilen Hydrogel-Beschichtungen bleibt bei den erfindungsgemäßen Hydrogel-Beschichtungen die Hydrophilie weitestgehend von der Schichtdicke unbeeinflusst. Das heißt die Schmutz-, Protein- und Zellabweisungseigenschaften bleiben schichtdickenunabhängig erhalten.

Weiterer Gegenstand der vorliegenden Erfindung sind die Mischungen der sternförmigen Präpolymere der allgemeinen Formel (II) wobei m und n unabhängig voneinander ≥ 1 sind und R² nicht für R¹ oder OH steht, wobei die Mischung: (a) mindestens ein sternförmiges Präpolymer mit drei bis 5 hydrophilen Polymerarmen und (b) mindestens ein sternförmiges Präpolymer mit mindestens 6 hydrophilen Polymerarmen, vorzugsweise 6 bis 10 Polymerarmen und besonders bevorzugt 6 bis 8 Polymerarmen enthält. Besondere Ausführungsformen dieses Gegenstands sind in den Ansprüchen 34 bis 48 beschrieben.

Vorzugsweise enthält die erfindungsgemäße Mischung aus Komponente (a) und Komponente (b) beziehungsweise die in den erfindungsgemäßen Beschichtungen eingesetzte Mischung aus Komponente (a) und Komponente (b), bezogen auf die Summe der Gewichtsanteile aus Komponente (a) und Komponente (b) mindestens 50 Gew.-% an Komponente (b), besonders bevorzugt mindestens 60 Gew.-% an Komponente (b), ganz besonders bevorzugt 65 bis 95 Gew.- % an Komponente (b), wie 70 bis 90 Gew.-% an Komponente (b).

Zur Herstellung der Lösung des sternförmigen Präpolymeren für das erfindungsgemäße Verfahren zur Herstellung einer Beschichtung auf einem Substrat eignen sich generell alle Lösemittel, welche keine oder nur eine geringe Reaktivität gegenüber den reaktiven Endgruppen des sternförmigen Präpolymers aufweisen. Beispiele sind Wasser, Alkohole, Wasser/Alkoholmischungen, aprotisches Lösungsmittel oder Gemische derselben.

Beispiele für geeignete aprotische Lösungsmittel sind beispielsweise Ether und cyclische Ether wie Tetrahydrofuran (THF), Dioxan, Diethylether, tert.-Butylmethylether, aromatische Kohlenwasserstoffe wie Xylole und Toluol, Acetonitril, Propionitril und Mischungen dieser Lösungsmittel. Werden sternförmige Präpolymere mit OH-, SH-, Carboxyl-, (Meth)acryl- und Oxirangruppen oder ähnlichen Gruppen als Endgruppen eingesetzt sind auch protische Lösungsmittel wie Wasser oder Alkohole, beispielsweise Methanol, Ethanol, n-Propanol, 2-Propanol, n-Butanol und tert.-Butanol, sowie deren Mischungen mit aprotischen Lösungsmitteln geeignet. Werden sternförmige Präpolymere mit Isocyanat-Gruppen eingesetzt, so sind neben den vorgenannten aprotischen Lösungsmitteln auch Wasser und Mischungen von Wasser mit aprotischen Lösungsmitteln geeignet. Vorzugsweise ist das Lösungsmittel Wasser beziehungsweise eine Mischung von Wasser mit aprotischen Lösungsmitteln.

Geeignete Mengen der Mischungen aus (a) und (b) in den Applikationsmischungen, die im erfindungsgemäßen Verfahren zur Beschichtung verwendet werden, richten sich nach den Schichtdicken, die für die jeweilige Anwendung am besten geeignet sind. Häufig reichen Mengen von beispielsweise etwa 0,005 bis 50 Gew.-%, vorzugsweise 0,1 bis 10 Gew.-% aus. Je nach Affinität des Substrates und Art der Applikation können zudem ebenfalls sowohl Applikationsmischungen mit einem höherer oder auch mit niedrigerem Gehalt der erfindungsgemäßen Mischungen oder den in den erfindungsgemäßen Beschichtungen eingesetzten Mischungen eingesetzt werden. Die Applikationsmischungen können dabei beispielsweise auch die Form von Pasten oder Cremes besitzen.

Die Herstellung der erfindungsgemäßen sternförmigen Präpolymere der allgemeinen Formel (II), welche in den erfindungsgemäßen Beschichtungen und dem erfindungsgemäßen Verfahren zur Herstellung einer Beschichtung eingesetzt werden erfolgt durch Funktionalisierung geeigneter sternförmiger Präpolymer-Vorstufen in Analogie zu bekannten Funktionalisierungsverfahren des Standes der Technik.

Die Präpolymer-Vorstufen der erfindungsgemäßen Präpolymere sind auch ihrerseits sternförmige Präpolymere, die bereits die oben beschriebene sternförmige Struktur, das heißt wenigstens drei für sich gesehen wasserlösliche Polymerarme, aufweisen und die an den Enden der Polymerarme je eine geeignete funktionelle Gruppe R³ aufweisen, die in die vorgenannten reaktiven Gruppen B-R¹ bzw. B-R² umgewandelt werden kann. Die Präpolymer-Vorstufen der erfindungsgemäßen Präpolymere lassen sich durch die allgemeine Formel (III) als Z-(X-A-R³)ₘ₊ₙ darstellen, wobei Z, X, A, m und n die gleiche Bedeutung haben, wie die entsprechenden Reste und Indices der in den erfindungsgemäßen Mischungen eingesetzten sternförmigen Präpolymere und R³ eine funktionelle Gruppe darstellt, die in die vorgenannten reaktiven Gruppen B-R¹ bzw. B-R² umgewandelt werden kann.

Zu den möglichen funktionellen Gruppen R³ zählen beispielsweise an aliphatische oder aromatische Kohlenstoffatome gebundene OH-Gruppen, Thiol-Gruppen, primäre oder sekundäre Amingruppen und Halogen-Atome wie Chlor, Brom oder Iod. Eine besonders bevorzugte Vorstufe betrifft die primären und sekundären OH-Gruppen, die sogenannten sternförmigen Polyether-Polyole. Diese Präpolymer-Vorstufen werden durch Polymerisation der geeigneten Monomeren unter Verwendung von mehrfunktionalen kleinen Molekülen wie zum Beispiel Sorbitol als Initiator hergestellt und können gegebenenfalls weiter modifiziert werden, um an ihren Enden eine erfindungsgemäße Gruppe -R³ zu generieren. Aufgrund der statistischen Natur der Polymerisationsreaktion verstehen sich die oben genannten Angaben zu den Polymerarmen der erfindungsgemäßen Präpolymere, insbesondere bezüglich der Armlänge und Armanzahl (m+n) als ein statistisches Mittel.

Als Ausgangsstoffe zur Umwandlung der Endgruppen R³ der sternförmigen Präpolymer-Vorstufe in die Gruppen B-R¹ kommen in der Regel alle funktionellen Silan-Derivate in Frage, die eine funktionelle Gruppe aufweisen, welche gegenüber den Endgruppen der Präpolymer-Vorstufe reaktiv ist. Beispiele sind Amino-Silane wie (3-Aminopropyl)triethoxysilan und N-(2-Aminoethyl)(3-aminopropyl)trimethoxysilan, (Meth)acrylat-Silane wie (3-Methacryloxypropyl)trimethoxysilan, (Methacryloxymethyl)triethoxysilan (Methacryloxymethyl)methyldimethoxysilan und (3-Acryloxypropyl)trimethoxysilan, Isocyanato-Silane wie (3-Isocyanatopropyl)trimethoxysilan, (3-Isocyanatopropyl)triethoxysilan, (Isocyanatomethyl)methyl-Dimethoxysilan und (Isocyanatomethyl)trimethoxysilan, Aldehyde-Silane wie Triethoxysilylundecanal und Triethoxysilylbutyraldehyde, Epoxy-Silane wie (3-Glycidoxypropyl)trimethoxysilan, Anhydridsilane wie 3-(Triethoxysilyl)propylbernsteinsäureanhydrid, Halogen-Silane wie Chloromethyltrimethoxysilan, 3-Chloropropylmethyldimethoxysilan, Hydroxyl-Silane wie Hydroxymethyltrietoxysilane, sowie Tetraethylsilikat (TEOS), die kommerziell beispielsweise bei der Wacker Chemie GmbH (Burghausen), der Gelest, Inc. (Morrisville, USA) oder ABCR GmbH & Co. KG (Karlsruhe) erhältlich sind oder nach bekannten Verfahren hergestellt werden können. Besonders bevorzugt werden Isocyanato-Silane bzw. Anhydrid-Silane mit hydroxyterminierten (R³ = OH) sternförmigen Polymeren der allgemeinen Formel (III) umgesetzt. Bei einer vollständigen Umsetzung aller Hydroxyenden mit Isocyanatosilanen erhält man die in den erfindungsgemäßen Mischungen eingesetzten sternförmigen Präpolymere, welche ausschließlich Reste R¹ tragen. Die Gruppe B enthält in einem solchen Fall eine Urethangruppe sowie die Atomgruppe, die im Ausgangs-Isocyanatosilan zwischen der Isocyanatogruppe und der Silylgruppe steht. Bei einer vollständigen Umsetzung aller Hydroxyenden mit Anhydrid-Silanen, beispielsweise 3-(Triethoxysilyl)propylbernsteinsäureanhydrid, erhält man erfindungsgemäße sternförmige Präpolymere, welche ausschließlich Reste R¹ tragen. Die Gruppe B enthält in einem solchen Fall eine Estergruppe sowie die Atomgruppe, die im Ausgangs-Anhydridsilan zwischen der Anhydridgruppe und der Silylgruppe steht.

Als Ausgangsstoffe zur Umwandlung der Endgruppen R³ der sternförmigen Präpolymer-Vorstufe in die Gruppen B-R², vorzugsweise eine Isocyanat-Gruppe, kommen in der Regel alle Diisocyanate, sowohl aromatische, als auch aliphatische, in Frage. Bevorzugt sind Diisocyanate, deren Isocyanatgruppen sich in ihrer Reaktivität unterscheiden, insbesondere sind aliphatische und cycloaliphatische Diisocyanate wie Isophorondiisocyanat (IPDI) bevorzugt. Bei der Umsetzung hydroxyterminierter sternförmiger Präpolymere mit Diisocyanaten bilden sich ebenfalls Urethangruppen im Rest B. Der Rest "B" kann innerhalb der erfindungsgemäßen sternförmigen Präpolymere jedoch in jedem der m+n Polymerarme eine unterschiedliche Bedeutung besitzen.

Werden erfindungsgemäße sternförmige Präpolymere der allgemeinen Formel (II) hergestellt, die sowohl B-R¹-als auch B-R²-Gruppen tragen, so wird vorzugsweise so vorgegangen, dass wie oben beschrieben zunächst B-R¹-Gruppen eingeführt werden, jedoch nicht alle R³-Gruppen im sternförmigen Präpolymer der allgemeinen Formel (III) umgesetzt werden. Auf diese Weise erhält man bereits sternförmige Präpolymere, die sowohl -R¹- als auch -R²-Gruppen tragen, hierbei handelt es sich um den besonderen Fall, in dem -R² gleich -R³ ist. So erhält man beispielsweise bei einer teilweisen Umsetzung aller Hydroxyenden mit Isocyanatosilanen erfindungsgemäße sternförmige Präpolymere, welche sowohl Reste R¹, also Silyl-gruppen, als auch OH-Gruppen (R²=R³) tragen. In einem weiteren Schritt können die restlichen oder ein Teil der restlichen R³-Gruppen - wie beschrieben - zu Resten R² beziehungsweise B-R² modifiziert werden. Beispielsweise erhält man, wenn -R² eine (Meth)acrylatgruppen darstellt, durch Veresterung der restlichen OH-Gruppen mit (Meth)acrylsäureanhydrid. In den meisten Fällen gelingt dies auch in einer umgekehrten Reaktionsfolge, d.h., man kann die Gruppe -R³ der sternförmigen Präpolymere zunächst in -R² umwandeln und dann mit einem funktionellen Alkoxysilan umsetzen, um die Gruppe -R¹ einzuführen.

Weiterer Gegenstand der vorliegenden Erfindung sind Derivate von erfindungsgemäßen Mischungen, die durch Reaktion der Gruppen R¹ und/oder R² mit den oben genannten Entites erhalten werden und in den Ansprüchen 48 und 49 beansprucht sind.

Neben den erfindungsgemäßen Mischungen sternförmiger Präpolymere des Anspruchs 33 können auch andere sternförmige Präpolymere zur Bildung der erfindungsgemäßen Beschichtungen eingesetzt werden, solange sie die erfindungsgemäßen Bedingungen, wie sie in Anspruch 1 festgelegt sind, erfüllen.

In den einfachsten Ausführungsformen werden freilich nur die Minimalanforderungen an die erfindungsgemäßen Beschichtungen erfüllt. So sind beispielsweise Mischungen sternförmiger Präpolymere, bei welchen die Silyl-Gruppen tragenden Moleküle über Diisocyanate angeknüpft werden, schlechter geeignet, gleichmäßig dichte Beschichtungen zu bilden als Mischungen sternförmiger Präpolymere der allgemeinen Formel (II), in welcher B maximal eine Urethan- oder Harnstoffbindung enthält. Gerade besonders dichte Schichten erlauben es, die Substrate vor einem sehr viel breiteren Spektrum an Verschmutzungen zu schützen.

Aus der Literatur bekannte sternförmige Präpolymere, können nur unter den oben genannten Voraussetzungen in den erfindungsgemäßen Beschichtungen und im erfindungsgemäßen Beschichtungsverfahren eingesetzt werden.

Die EP 0931800 A1 betrifft ein silyliertes Polyurethan, das durch erste Umsetzung eines Polyols mit stöchiometrisch unterschüssigem Diisocyanat und anschließender Umsetzung des erhaltenen Isocyanat-Hydroxy-Polyols mit Isocyanatsilanen hergestellt wurde.

Die US 2003 0153712 A1 beschreibt ein Polyurethan-Präpolymer mit terminalen Alkoxysilan- und Hydroxy-Gruppen. Zur Herstellung wurde ein Polyether-Diol erst mit stöchiometrisch unterschüssigem Diisocyanat umgesetzt, die erhaltene Isocyanat-Hydroxy-Verbindung wurde dann weiter mit einem Aminosilan zur Einführung der Silyl-Gruppen umgesetzt.

Aus der EP 0935627 A1 ist ein auf Polyether basierendes sternförmiges Präpolymer bekannt, das an seinen freien Enden zwei unterschiedlich reaktive funktionelle Gruppen R¹ und R² trägt. Hierbei steht R¹ für eine Isocyanat-Gruppe, während R² eine unter normalen Bedingungen mit R¹ nichtreaktive Gruppe darstellt. Zur Herstellung solcher Präpolymere wurden alle OH-Gruppen der Polyether-Polyole zunächst mit stöchiometrisch überschüssigen Diisocyanaten umgesetzt und die so erhaltenen NCO-Präpolymere weiter mit einer stöchiometrisch unterschüssigen bifunktionalen Verbindung, die eine endständige Isocyanat-reaktive Gruppe und eine andere endständige nicht isocyanat-reaktive Gruppe trägt, behandelt. Solche Präpolymere können beispielsweise zur Beschichtung von Oberflächen verwendet werden.

Aus der US 20020042471 A1 und der US 20030027921 A1 sind Präpolymere mit 2 bis 6 Isocyanat-Gruppen bekannt, die mit einer stöchiometrisch unterschüssigen Menge Aminosilan weiter modifiziert wird. Die erhaltenen Präpolymere haben sowohl NCO- als auch Silan-Gruppen und werden zusammen mit einem Polyol als Beschichtungsmaterial verwendet.

In der US 6423661 B1 und WO 9955765 A1 wird ein auf Polyether basierendes silyl-terminiertes Präpolymer beschrieben. Zur Herstellung wurden alle OH-Gruppen eines Polyether-Polyols mit einem stöchiometrisch überschüssigen Isocyanat-Silan umgesetzt. Solche Präpolymere sollen als Klebstoffe verwendet werden.

Eine ähnliche Verbindung, ein sechsarmiges silyl-terminiertes Polyethylenglykol, wurde in der US 2004 0096507 A1 beschrieben.

Die unter Verwendung von erfindungsgemäßen Mischungen aus (a) und (b) oder den in Beschichtungen nach Anspruch 1 eingesetzten Mischungen aus (a) und (b) hergestellten erfindungsgemäßen Hydrogel-Beschichtungen verhindern wirksam die Adsorption von Proteinen und Zellen und können für viele Anwendungen, wie beispielsweise im Hygiene- und bioanalytischen Bereich eingesetzt werden. Daher ist auch eine derartige Verwendung unter anderem Gegenstand der vorliegenden Erfindung.

Weiterer Gegenstand der vorliegenden Erfindung ist die Verwendung der erfindungsgemäßen Mischungen sternförmiger Präpolymere, der Derivate davon und/oder der in den erfindungsgemäßen Beschichtungsmittel eingesetzten Mischungen sternförmiger Präpolymere und/oder sternförmiger Präpolymer-Nanoteilchen-Komplexe in Anti-Soiling-Mitteln zur temporären oder permanenten Ausrüstung von Oberflächen. Wesentliche Voraussetzung hierfür ist das hydrophile Oberflächenverhalten bei gleichzeitig geringer Kontaktwinkelhysterese. Die Hydrophilie der Oberfläche erschwert einerseits die Adsorption und Anhaftung protein- und fetthaltiger Anschmutzungen und andererseits erlaubt eine effiziente Benetzung mit Reinigungsmittel, wodurch Verunreinigungen leichter als bei hydrophoben Oberflächen vom Substrat getrennt werden können. Die durch die geringe Kontaktwinkelhysterese gekennzeichnete Entnetzung, bzw. das vollständige Ablaufen der Reinigungslösung verhindert darüber hinaus effektiv die Wiederanlagerung von Schmutz auf den frisch gereinigten Oberflächen.

Eine weitere erfindungsgemäße Verwendung der erfindungsgemäßen Mischungen sternförmiger Präpolymere, der Derivate davon und/oder der in den erfindungsgemäßen Beschichtungsmittel eingesetzten Mischungen sternförmiger Präpolymere und/oder sternförmiger Präpolymer-Nanoteilchen-Komplexe besteht in deren Verwendung als Additive in Reinigungsmitteln und Waschmitteln für harte und weiche Oberflächen, wie sie beispielsweise im Sanitär- oder Küchenbereich (maschinelle und Hand-Geschirrspülmittel) verwendet werden, um eine Anschmutzung oder Wiederanschmutzung zu verhindern oder zu reduzieren, in Haarpflegemitteln, Textilbehandlungsmitteln, Wand-, Fassaden- und Fugenbehandlungsmitteln, in Mitteln zur Behandlung von Fahrzeugen, wie Automobilen, Flugzeugen, Schiffen oder Booten (Anti-Fouling) und in Mitteln zur Innen- und Außenbeschichtung von Behältern um beispielsweise ein verlustfreies Entleeren der Behälter zu ermöglichen, oder in Mitteln zur Beschichtung von Bioreaktoren und Wärmeaustauschern, um beispielsweise das Anhaften von Mikroorganismen zu verhindern.

Eine weitere erfindungsgemäße Verwendung der erfindungsgemäßen Mischungen sternförmiger Präpolymere, der Derivate davon und/oder der in den erfindungsgemäßen Beschichtungsmittel eingesetzten Mischungen sternförmiger Präpolymere und/oder sternförmiger Präpolymer-Nanoteilchen-Komplexe stellt die Verwendung in Beschichtungen zur Beeinflussung des Aufwachsens beziehungsweise der Auf-Kristallisation von Feststoffen auf der Oberfläche dar. Durch ihre dichte Struktur, ihre Hydrophilie sowie ihre leichte chemische Funktionalisierbarkeit - beispielsweise durch Entities - kann mit den erfindungsgemäßen Hydrogelschichten im Prinzip die biologische Situation bei Biomineralisationvorgängen nachgestellt werden. Als Beispiel für einen typischen Biomineralisationsvorgang sei die Bildung von Muschelschalen aus Calciumcarbonat genannt, die durch spezifisch strukturierte und funktionalisierte hydrophile Polymerschichten gesteuert wird. Die Natur lehrt dabei, dass durch die Einzelheiten der chemischen Struktur solcher hydrophilen Polymere, das Wachstum von Festkörpern aus Lösung entweder gefördert und/oder gesteuert oder aber verhindert werden kann. Als ein technisch und ökonomisch relevanter Wachstumsprozess ist hierbei Kalkkristallisation auf Oberflächen zu nennen. Durch die erfindungsgemäßen Hydrogelschichten, gegebenenfalls durch Zusatz geeigneter Entities, kann das Aufwachsen von Kalk verhindert werden. Die Kalkabscheidung wird über die hier geschilderte Substratwirkung hinaus auch dadurch verhindert, dass Wasser wie oben erwähnt auf den beschichteten Oberflächen entnetzt und somit aufgrund dieses einfachen physikalischen Effekts eine Kristallisation verhindert wird. Die Antikalkbeschichtung auf Hydrogelbasis kann dabei permanenter oder aber auch temporärer Natur sein.

Durch einen Einbau geeigneter Entities kann allerdings nicht nur die Aufwachsung von Festkörpern verhindert werden, sondern im Gegenteil auch gezielt das gegebenenfalls auch kristallographisch orientierte Aufwachsen von Feststoffen auf Substraten, vorzugsweise von solchen mit technisch nutzbaren Funktionalitäten, induziert werden. Durch die genauen Details der chemischen Zusammensetzung der Beschichtung, insbesondere durch die Entities ist somit eine generelle Steuerung des Aufwachsens von Feststoffen möglich.

Eine weitere erfindungsgemäße Verwendung der erfindungsgemäßen Mischungen sternförmiger Präpolymere, der Derivate davon und/oder der in den Mischungen erfindungsgemäßer Beschichtungsmittel eingesetzten sternförmigen Präpolymere und/oder sternförmigen Präpolymer-Nanoteilchen-Komplexe ist bei der Herstellung von Mikroarrays oder Sensoren für bioanalytische Zwecke oder zur Beschichtung mikrofluidischer Bauteile oder zur Beschichtung von Mikrokanülen und Kapillarsystemen, beispielsweise für die Einbringung von genetischem Material in Zellen gegeben. Hierbei erlaubt die Hydrogel-Beschichtung einerseits eine selektive Kopplung von Biomolekülen an die Beschichtung, wenn diese beispielsweise Rezeptoren als Entity gebunden aufweisen, andererseits zeichnet sie sich durch eine besonders geringe Affinität gegenüber unspezifischer Bindung von Biomolekülen aus. Daher sind die Hydrogel-Beschichtungen als Beschichtungsgrundlage von Substraten für Bioanalysesysteme besonders geeignet.

Gegenstände der vorliegenden Erfindung sind daher auch Anti-Soiling-Mittel, Reinigungsmittel und Waschmittel für harte und weiche Oberflächen, Haarpflegemittel, Textilbehandlungsmittel, Wand-, Fassaden- und Fugenbehandlungsmittel, Mitteln zur Behandlung von Fahrzeugen, Mittel zur Innen- und Außenbeschichtung von Behältern, Bioreaktoren und Wärmeaustauschern enthaltend die erfindungsgemäßen Mischungen der sternförmigen Präpolymere beziehungsweise der in den Beschichtungen nach Anspruch 1 eingesetzten Mischungen.

Eine weitere erfindungsgemäße Verwendung der Mischungen sternförmiger Präpolymere, der Derivate davon und/oder der in den erfindungsgemäßen Beschichtungsmittel eingesetzten Mischungen sternförmiger Präpolymere und/oder sternförmiger Präpolymer-Nanoteilchen-Komplexe ist die Ausrüstung von Oberflächen mit veränderten, insbesondere verringerten Reibungseigenschaften. Trägt man beispielsweise die Beschichtungen auf Textilien auf, so wird damit ein angenehmerer Griff erzeugt, bei der Anwendung auf Haaren wird beispielsweise die Kämmbarkeit verbessert.

Auch die Verwendung dieser Mischungen zur Verminderung statischer elektrischer Aufladungen ist Gegenstand dieser Erfindung. Stabile hydrophile Beschichtungen beispielsweise auf Haar verhindern über längere Zeiträume negative elektrostatische Effekte. Dasselbe gilt natürlich auch für Textilien.

Eine weitere erfindungsgemäße Verwendung der Mischungen sternförmiger Präpolymere, der Derivate davon und/oder der in den erfindungsgemäßen Beschichtungsmittel eingesetzten Mischungen sternförmiger Präpolymere und/oder sternförmiger Präpolymer-Nanoteilchen-Komplexe besteht darin, durch die Hydrogelbeschichtung auf Textilien entweder aufgrund der Hydrogelstruktur selbst oder aber durch zusätzliche Funktionalitäten, die vorzugsweise durch die oben genannten Entities eingebracht werden, Farbstoffe auf der Faser zu fixieren bzw. zurückzuhalten. Auf diese Weise wird ein Farbschutzeffekt erzielt, der sich beispielsweise in einem No-Sort-Waschmittel, das heißt einem Waschmittel mit dem bunte und weiße Wäschen gewaschen werden kann, nutzen lässt.

### BEISPIELE

Die im Beispielteil angegebenen Molekulargewichte sind zahlenmittlere Molekulargewichte der Ausgangsverbindungen (Polyether-Polyole), die zur Herstellung der Präpolymere eingesetzt wurden. Das zahlenmittlere Molekulargewicht der Polyole lässt sich durch Endgruppenbestimmung rechnerisch aufgrund der Kenntnis der Funktionalität der Verbindungen oder der Funktionalität der Mischungskomponenten und der OH-Zahl der Verbindung oder der Mischung (ermittelt nach DIN 53240) bestimmen. Für den Fall, dass als Ausgangsverbindungen andere Verbindungen anstelle der Polyole verwendet werden, ist deren zahlenmittleres Molekulargewicht maßgeblich. So kann beispielsweise das zahlenmittlere Molekulargewicht von Aminen über eine Endgruppenbestimmung durch potentiometrische Titration nach der DIN 16945 ermittelt werden.

Herstellung geeigneter sternförmiger Präpolymere:

### Beispiel 1: Drejarmiger Triethoxysilvl-terminierter Polyether (PP1):

Das verwendete Polyether-Polyol ist ein 3-armiges statistisches Poly(ethylenoxid-co-propylenoxid) mit einem EO/PO-Verhältnis von 75/25 und mit einem zahlenmittleren Molekulargewicht von ca. 5000 g/mol, das von der Firma DOW Chemicals bezogen wurde (Voranol® CP 1421). Vor der Umsetzung wurde das Polyol im Vakuum unter Rühren für 1 h auf 80 °C erwärmt.

Zum getrockneten Polyether-Polyol (2,04 g, 0,41 mmol) wurde (3-Isocyanatopropyl)triethoxysilan (317 mg, 1,0 eq.) langsam zugegeben. Die Reaktionsmischung wurde weiter unter Schutzgas bei 100 °C für 2 Tage gerührt, bis die Schwingungsbande der NCO-Gruppe bei einer IR-Messung verschwunden ist. Man erhält ein Produkt, bei welchem jeweils eine Triethoxysilyl-Gruppe an den freien Enden der Polymerarme des sternförmigen Präpolymers vorhanden ist. Das Produkt ist eine farblose, viskose Flüssigkeit.

### Beispiel 2: Sechsarmiger Triethoxysilyl-terminierter Polyether (PP2):

Das verwendete Polyether-Polyol ist ein 6-armiges statistisches Poly(ethylenoxid-co-propylenoxid) mit einem EO/PO-Verhältnis von 80/20 und mit einem Molekulargewicht von 12000 g/mol, das durch anionische ringöffnende Polymerisation von Ethylenoxid und Propylenoxid unter Verwendung von Sorbitol als Initiator hergestellt wurde. Vor der Umsetzung wurde das Polyol im Vakuum unter Rühren für 1 h auf 80 °C erwärmt.

Eine Lösung von Polyether-Polyol (3 g, 0,25 mmol), Triethylendiamin (9 mg, 0,081 mmol) und Dibutylzinndilaureat (9 mg, 0,014 mmol) in 25 ml wasserfreiem Toluol wurde vorgelegt, dazu wurde eine Lösung von (3-Isocyanatopropyl)triethoxysilan (0,6 ml, 2,30 mmol) in 10 ml wasserfreiem Toluol tropfenweise zugegeben. Die Lösung wurde weiter bei 50°C über Nacht gerührt. Nach dem Entfernen des Toluols unter Vakuum wurde das Rohprodukt wiederholt mit wasserfreiem Ether gewaschen. Nach dem Vakuumtrocknen erhielt man das Produkt, welches jeweils eine Triethoxylsilyl-Gruppe an den freien Enden der Polymerarme des sternförmigen Präpolymers aufweist, als eine farblos viskose Flüssigkeit. IR (Film, cm⁻¹): 3349 (m, -CO-NH-), 2868 (s, -CH₂-, - CH₃), 1719 (s, -C=O), 1456 (m, -CH₂-, -CH₃), 1107 (s, -C-O-C-), 954 (m, -Si-O-). ¹H-NMR (Benzol- d₆, ppm): 1,13 (d,-CH₃ von Polymerarmen), 1,21 (t, -CH₃ von Silan-Endgruppen), 3,47 (s, -CH₂ von Polymerarmen), 3,74 (q, -CH₂ von Silan-Endgruppen).

### Beispiel 3: Sechsarmiger triethoxysilyl-hydroxy-terminierter Polyether (PP3):

Analog zu Beispiel 2 wurde eine Lösung von Polyether-Polyol (10 g, 0,83 mmol), Triethylendiamin (30 mg, 0,27 mmol) und Dibutylzinndilaureat (30 mg, 0,048 mmol) in 50 ml wasserfreiem Toluol vorgelegt, dazu wurde eine Lösung von (3-Isocyanatopropyl)triethoxysilan (0,65 ml, 2,49 mmol) in 15 ml wasserfreiem Toluol tropfenweise zugegeben. Die Lösung wurde weiter bei 50 °C über Nacht gerührt. Nach dem Entfernen des Toluols unter Vakuum wurde das Rohprodukt durch IR analysiert. Die Ergebnisse zeigten, dass die typischen Schwingungen der NCO-Gruppe bei ca. 2270 cm⁻¹ vollständig verschwanden und einhergehend verminderte OH-Schwingungen bei ca. 3351 cm⁻¹ zu sehen waren, was darauf hindeutet, dass die Isocyanatosilane-Moleküle über eine Urethan-Bindung erfolgreich an den Enden des Polyols angeknüpft sind. Das Rohprodukt wurde dann wiederholt mit wasserfreiem Ether gewaschen. Nach dem Vakuumtrocknen erhielt man das Produkt, das Triethoxylsilyl- und Hydroxy-Gruppen mit einem statistischen Verhältnis von 3/3 an den freien Enden der Polymerarme der sternförmigen Präpolymere aufweist, als eine farblos viskose Flüssigkeit. IR (Film, cm⁻¹): 3511, (m, -OH), 3351 (m, -CO-NH-). 2868 (s, -CH₂-, -CH₃), 1720 (s, -C=O), 1456 (m, -CH₂-, -CH₃), 1112 (s, -C-O-C-), 953 (m, -Si-O-). ¹H-NMR (Benzol-d₆, ppm): 1,08-1,17 (m, -CH₃ von Polymerarmen und -CH₃ von Silan-Endgruppen), 3,47 (s, -CH₂ von Polymerarmen), 3,74 (q, -CH₂ von Silan-Endgruppen).

### Beispiel 4: Sechsarmiger triethoxysilyl-hydroxy-terminierter Polyether (PP4):

Analog zu Beispiel 2 wurde eine Lösung von Polyether-Polyol (10 g, 0,83 mmol), Triethylendiamin (30 mg, 0,27 mmol) und Dibutylzinndilaureat (30 mg, 0,048 mmol) in 50 ml wasserfreiem Toluol vorgelegt. Dazu wurde eine Lösung von (3-Isocyanatopropyl)triethoxysilan (0,22 ml, 0,84 mmol) in 15 ml wasserfreiem Toluol tropfenweise gegeben. Die Lösung wurde weiter bei 50 °C über Nacht gerührt. Nach dem Entfernen des Toluols unter Vakuum wurde das Rohprodukt wiederholt mit wasserfreiem Ether gewaschen. Nach dem Vakuumtrocknen erhielt man das Produkt, welches Triethoxylsilyl- und Hydroxy-Gruppen mit einem statistischen Verhältnis von 1/5 an den freien Enden der Polymerarme der sternförmigen Präpolymere aufweist, als eine farblos viskose Flüssigkeit. IR (Film, cm⁻¹): 3494, (m, -OH), 3346 (w, -CO-NH-), 2868 (s, -CH₂-, -CH₃), 1722 (m, - C=O), 1456 (m, -CH₂-, -CH₃), 1112 (s, -C-O-C-), 952 (m, -Si-O-). ¹H-NMR (Benzol-d₆, ppm): 1,08-1,18 (m, -CH₃ von Polymerarmen und -CH₃ von Silan-Endgruppen), 3,49 (s, -CH₂ von Polymerarmen), 3,75 (q, -CH₂ von Silan-Endgruppen).

Analog zu Beispiel 3 und 4 wurden weitere triethoxysilyl-hydroxy-terminierte Polyether hergestellt:
Beispiel 5: Triethoxysilyl- und Hydroxy-Gruppen (Verhältnis Triethoxysilyl/OH = 2/4; PP5): Farblose viskose Flüssigkeit. IR (Film, cm⁻¹): 3496, (m, -OH), 3351 (w, -CO-NH-), 2869 (s, -CH₂-, -CH₃), 1721 (m, -C=O), 1459 (m, -CH₂-, -CH₃), 1107 (s, -C-O-C-), 953 (m, -Si-O-). ¹H-NMR (Benzol-d₆, ppm): 1,05-1,16 (m, -CH₃ von Polymerarmen und - CH₃ von Silan-Endgruppen), 3,47 (s, -CH₂ von Polymerarmen), 3,74 (q, -CH₂ von Silan-Endgruppen).
Beispiel 6: Triethoxysilyl- und Hydroxy-Gruppen (Verhältnis Triethoxysityl/OH = 5/1: PP6): Farblose viskose Flüssigkeit. IR (Film, cm⁻¹): 3512, (m, -OH), 3351 (w, -CO-NH-), 2867 (s, -OH₂-, -CH₃), 1715 (m, -C=O), 1457 (m, -CH₂-, -CH₃), 1116 (s, -C-O-C-), 952 (m, -Si-O-). ¹H-NMR (Benzol-d₆, ppm): 1,08-1,17 (m, -CH₃ von Polymerarmen und - CH₃ von Silan-Endgruppen), 3,47 (s, -CH₂ von Polymerarmen), 3,74 (q, -CH₂ von Silan-Endgruppen).
Beispiel 7: Triethoxysilyl- und Hydroxy-Gruppen (Verhältnis Triethoxysilyl/OH = 4/2: PP7): Farblose viskose Flüssigkeit. IR (Film, cm⁻¹): 3513, (m, -OH), 3351 (w, -CO-NH-), 2867 (s, -CH₂-, -CH₃), 1721 (m, -C=O), 1455 (m, -CH₂-, -CH₃), 1106 (s, -C-O-C-), 954 (m, -Si-O-). ¹H-NMR (Benzol-d₆, ppm): 1,05-1,16 (m, -CH₃ von Polymerarmen und - CH₃ von Silan-Endgruppen), 3,46 (s, -CH₂ von Polymerarmen), 3,73 (q, -CH₂ von Silan-Endgruppen).

### Beispiel 8:Sechsarmiger triethoxysilyl-isocyanat-terminierter Polyether (PP8):

Eine Mischung des Produkts aus Beispiel 2 (4 g, 0,32 mmol), Isophorondiisocyanat, (IPDI, 3,2 ml, 15,1 mmol) und 7 ml wasserfreiem Toluol wurde bei 50 °C für 48 Stunden gerührt. Nach dem Entfernen des Toluols unter Vakuum wurde das Rohprodukt wiederholt mit wasserfreiem Ether gewaschen. Nach dem Vakuumtrocknen erhielt man das Produkt, das Triethoxylsilyl- und Isocyanat-Gruppen mit einem statistischen Verhältnis von 3/3 an den freien Enden der Polymerarme der sternförmigen Präpolymere aufweist, als eine farblose viskose Flüssigkeit. IR (Film, cm⁻¹): 3335 (w, -CO-NH-). 2869 (s, -CH₂-, -CH₃), 2266 (s, -NCO), 1717 (s, -C=O), 1458 (m, -CH₂-, -CH₃), 1111 (s, -C-O-C-), 953 (m, -Si-O-). ¹H-NMR (Benzot-d₆. ppm): 1,11-1,18 (m, -CH₃ von Polymerarmen und -CH₃ von Silan-Endgruppen), 3,49 (s, -CH₂ von Polymerarmen), 3,75 (q, -CH₂ von Silan-Endgruppen).

### Beispiel 9: Sechsarmiger triethoxysilyl-isocyanat-terminierter Polyether (PP9):

Eine Mischung des Produkts aus Beispiel 3 (4,7 g, 0,38 mmol), Isophorondiisocyanat, (IPDI, 5,65 ml, 26,7 mmol) und 5 ml wasserfreiem Toluol wurde bei 50 °C für 48 Stunden gerührt. Nach dem Entfernen des Toluols unter Vakuum wurde das Rohprodukt wiederholt mit wasserfreiem Ether gewaschen. Nach dem Vakuumtrocknen erhielt man das Produkt, welches Triethoxylsilyl- und Isocyanat-Gruppen mit einem statistischen Verhältnis von 1/5 an den freien Enden der Polymerarme der sternförmigen Präpolymere aufweist, als eine farblose viskose Flüssigkeit. IR (Film, cm⁻¹): 3335 (w, -CO-NH-), 2869 (s, -CH₂-, -CH₃), 2266 (s, -NCO), 1717 (s, -C=O), 1458 (m, -CH₂-, -CH₃), 1112 (s, -C-O-C-), 952 (m, -Si-O-). ¹H-NMR (Benzol-d₆. ppm): 1,11-1,18 (m, -CH₃ von Polymerarmen und -CH₃ von Silan-Endgruppen), 3,48 (s, -CH₂ von Polymerarmen), 3,75 (q, -CH₂ von Silan-Endgruppen).

Analog zu Beispiel 8 und 9 wurden weitere triethoxysilyl-isocyanat-terminierte Polyether hergestellt:
Beispiele 10: Triethoxysilyl- und Isocyanat-Gruppen (Verhältnis Triethoxysilyl/NCO = 2/4: PP10): Farblose viskose Flüssigkeit. IR (Film, cm⁻¹): 3335 (w, -CO-NH-), 2869 (s, -CH₂-, -CH₃), 2265 (s, -NCO), 1718 (s, -C=O), 1460 (m, -CH₂-. -CH₃), 1112 (s, -C-O-C-), 952 (m, -Si-O-). ¹H-NMR (Benzol-d₆, ppm): 1,11 -1,17 (m, -CH₃ von Polymerarmen und - CH₃ von Silan-Endgruppen), 3,48 (s, -CH₂ von Polymerarmen). 3,75 (q, -CH₂ von Silan-Endgruppen).
Beispiel 11: Triethoxysilyl- und Isocyanat-Gruppen (Verhältnis Triethoxysilyl/NCO = 5/1 PP11): farblose viskose Flüssigkeit. IR (Film, cm⁻¹): 3342 (w, -CO-NH-), 2869 (s, -CH₂-, -CH₃), 2265 (s, - NCO), 1719 (s, -C=O), 1460 (m, -CH₂-, -CH₃), 1114 (s, -C-O-C-), 954 (m, -Si-O-). ¹H-NMR (Benzol-d₆, ppm): 1,09-1,17 (m, -CH₃ von Polymerarmen und - CH₃ von Silan-Endgruppen), 3,48 (s, -CH₂ von Polymerarmen), 3,75 (q, -CH₂ von Silan-Endgruppen).
Beispiel 12: Triethoxysilyl- und Isocyanat-Gruppen (Verhältnis Triethoxysilyl/NCO = 4/2: PP12): farblose viskose Flüssigkeit. IR (Film, cm⁻¹): 3340 (w, -CO-NH-), 2869 (s, -CH₂-, -CH₃), 2265 (s, - NCO), 1719 (s, -C=O), 1459 (m, -CH₂-, -CH₃), 1109 (s, -C-O-C-), 953 (m, -Si-O-). ¹H-NMR (Benzol-d₆, ppm): 1,12-1,17 (m, -CH₃ von Polymerarmen und - CH₃ von Silan-Endgruppen), 3,49 (s, -CH₂ von Polymerarmen), 3,75 (q, -CH₂ von Silan-Endgruppen).
Beispiel 13: Sechsarmiger Triethoxysilyl-terminierter Polyether (PP13): Das verwendete Polyether-Polyol ist ein 6-armiges statistisches Poly(ethylenoxid-co-propylenoxid) mit einem EO/PO-Verhältnis von ca. 80/20 und mit einem zahlenmittleren Molekulargewicht von ca. 3000 g/mol, das durch anionische ringöffnende Polymerisation von Ethylenoxid und Propylenoxid unter Verwendung von Sorbitol als Initiator hergestellt wurde. Vor der Umsetzung wurde das Polyether-Polyol im Vakuum unter Rühren für 1 h auf 80 °C erwärmt.

Zu dem getrockneten Polyether-Polyol (20 g, 6,67 mmol) wurde Dibutylzinndilaureat (2 mg, 0,01%) und (3-Isocyanatopropyl)triethoxysilan (9,5 g, 1,0 eq.) langsam zugegeben. Die Reaktionsmischung wurde weiter unter Schutzgas bei Raumtemperatur für 2 Tage gerührt, bis die NCO-Band bei IR-Messung verschwunden ist. Man erhielt ein Produkt, welches jeweils eine Triethoxylsilyl-Gruppe an den freien Enden der Polymerarme der sternförmigen Präpolymere aufweist, als eine farblos viskose Flüssigkeit.

### Beispiel 14: Mischung aus dreiarmigem Triethoxysityl-terminiertem Polyether und achtarmigem Triethoxysilyl-terminiertem Polyether (PP14):

Die verwendete Polyether-Polyol-Mischung besteht aus einem 3-armigen statistischen Poly(ethylenoxid-co-propylenoxid) (Glycerin-gestartet) und einem 8-armigen Polyether-Polyol (Rohrzucker-gestartet). Die Polymerarme sind jeweils statistische Poly(ethylenoxid-co-propylenoxide) mit einem EO/PO-Verhältnis von 75/25. Die OH-Funktionalität beträgt durchschnittlich 6,9 (durch Endgruppenbestimmung ermittelt), wodurch sich ein durchschnittliches zahlenmittleres Molekulargewicht von ca. 12000 g/mol ergibt. Es ergibt sich ein Verhältnis von 78 Gew.-% an 8-armigem Polyether-Polyol zu 22 Gew.-% an 3-armigem Polyether-Polyol. Das Polyether-Polyol-Gemisch wurde von der Firma DOW Chemicals bezogen (Voranol® 4053). Vor der Umsetzung wurde das Polyether-Polyol im Vakuum unter Rühren für 1 h auf 80 °C erwärmt.

Zum getrockneten Polyether-Polyol (209 g, 16,9 mmol) wurde Dibutylzinndilaureat (20,9 mg, 0,01 %) und (3-Isocyanatopropyl)triethoxysilan (30,3 g, 1,0 eq.) langsam zugegeben. Die Reaktionsmischung wurde weiter unter Schutzgas bei 100 °C für 2 Tage gerührt, bis die Schwingungsbande der NCO-Gruppe bei einer IR-Messung verschwunden ist. Man erhält ein Produkt, bei welchem jeweils eine Triethoxysilyl-Gruppe an den freien Enden der Polymerarme der beiden sternförmigen Präpolymere vorhanden ist. Das Produkt ist eine farblose, viskose Flüssigkeit.

Herstellung der Hydrogel-Beschichtungen:
Zur Herstellung der erfindungsgemäßen Hydrogel-Beschichtungen und Vergleichs-Beschichtungen wurden ein Teil der synthetisierten sternförmigen Präpolymere einzeln bzw. erfindungsgemäß gemischt eingesetzt. Das eingesetzte Präpolymer beziehungsweise die eingesetzte Mischung von verschiedenen Präpolymeren (5 Gew.-%) wurde mit Wasser (2,5 Gew.-%) und Essigsäure (2,5 Gew.-%) in Ethanol bei Raumtemperatur für 1-2 Tage gerührt. Anschließend wurden diese Lösungen mit Wasser zwanzigfach verdünnt und auf gereinigte Glasoberflächen (Objektträger, bezogen von der Karl Roth GmbH, "ready-to-use") gesprüht. Nach dem Abspülen mit fließendem Wasser erhielt man eine erfindungsgemäße Beschichtung.

Untersuchungen an Hydrogel-Beschichtungen:

### Messung des statischen Wasserkontaktwinkels und der Kontaktwinkelhysterese

Die Messungen wurden mit einem Kontaktwinkelmessgerät der Firma Data Physics GmbH durchgeführt (Gerätetyp: OCA20; elektronische Kippvorrichtung TBU90E; elektronisches Spritzenmodul ES; Software: SCA inkl. Software-Update für SCA-Module (Version 3.11.6 Build 155)).

Das Gerät wird vor der Messung mittels der Kalibrierungsautomatik des Geräts kalibriert. Mittels des Spritzenmoduls wird ein Tropfen destilliertes Wasser (15 µl) auf die zu messende Oberfläche des Objektträgers aufgebracht. Der Kippwinkel beträgt 0°, d.h. die zu messende Fläche ist waagerecht. Es erfolgt eine Aufnahme des Tropfen mit einer Videokamera. Am Einzelbild wird mittels der Software eine Tangente am Tropfenquerschnitt an dem Punkt angelegt, an dem der Tropfen die Oberfläche berührt. Der sich ergebende Winkel zwischen der Tangente und der zu messenden Oberfläche wird als statischer Kontaktwinkel bezeichnet (Sessile-Drop-Verfahren).

Anschließend wird die Probe samt Probentisch und der Kamera bei der langsamsten Geschwindigkeit, die das Gerät zulässt (rechnerisch aus den Geräteangaben: 0,62 °/s) bis zu einem Winkel von 90° gekippt. Während dieses Vorgangs wird ein Video des Tropfens durch die Kamera mittels der Software aufgenommen, wobei der Kippwinkel zum Zeitpunkt der Aufnahme mitgespeichert wird. Sobald der Tropfen beginnt von der Oberfläche abzulaufen wird die Messung beendet. Mittels der Software kann anschließend im Video der Fortschreitwinkel (Winkel in Tropfenfließrichtung) und der Rückzugswinkel (auf der anderen Seite des Tropfens) mittels der Ellipsen-Methode der Messsoftware zum Zeitpunkt des beginnenden Ablaufens des Tropfens von der Oberfläche bestimmt werden. Die Differenz der beiden Winkel ist die Kontaktwinkelhysterese (Tilting-Plate-Verfahren).

### Schuhcreme-Test

Der "Schuhcremeschmutz" wurde folgendermaßen hergestellt: Eine Mischung aus Schwarzschuhcreme (6,5 Gew.%), Mazola-Öl (3,5 Gew.%), Bratensauce (26 Gew.%) und Leitungswasser (64 Gew.%) wurde bei 100°C für 2 min gekocht. Nach anschließendem Rühren für 20 min und dem Abkühlen auf Raumtemperatur wird der Schuhcremeschmutz erhalten. Die Testoberflächen wurden für 2 min in den Schuhcremeschmutz getaucht, nach Herausnehmen wurden die Testoberflächen bei Raumtemperatur für 1 min getrocknet und anschließend mit fließendem Wasser abgespült, bis der schwarze Schuhcremeschmutz vollständig von der Oberfläche entfernt ist. Die Menge und Verteilung der auf der Oberfläche verbleibenden Schmutzreste (weiße Fettschicht) wurde als Kriterium für den Easy-to-Clean-Effekt verwendet.

### IKW-Test

Die beschichtete Glasoberfläche wurde mit IKW-Ballastschmutz, hergestellt nach SÖFW-Journal, 1998,124,1029, überschichtet und über Nacht bei Raumtemperatur getrocknet, als Referenz dient eine nicht behandelte Glasoberfläche. Nach dem Trocknen wurden die Oberflächen mit fließendem Wasser abgewaschen. Die Menge und Verteilung der auf der Oberfläche verbleibenden Schmutzreste (weiße Fettschicht) wurde als Kriterium für den Easy-to-Clean-Effekt verwendet.

| Beschichtungen | Kontaktwinkel Θ_{statisch} (grad) | Hysterese (grad) | Schuhcremetest | IKW-Test |
|---|---|---|---|---|
| PP1 | 44 | 15 | ++ | ++ |
| PP2 | 41 | 9 | ++/+++ | ++/+++ |
| PP1/PP2 (1/4) | 42 | 13 | +++ | +++ |
| PP14 | 45 | 11 | +++ | +++ |

| | | | | |
|---|---|---|---|---|
| O = nicht besser als die Referenz (=unbeschichtet); + = mäßig besser als die Referenz; ++ = deutlich besser als die Referenz; +++ = sehr viel besser als die Referenz | | | | |

Das reine 3-armige sternförmige Präpolymer zeigt die schlechtesten Ergebnisse hinsichtlich der Kontaktwinkelhysterese, des Schuhcremetests und des IKW-Tests. Das reine 6-armige sternförmige Präpolymer zeigt zwar eine gute Hysterese, schneidet jedoch bezüglich des Schuhcremetests und des IKW-Tests nicht optimal ab. Nur die Mischungen aus 3-armigem und 3- armigem bzw. 3-armigem und 8-armigem Sternpolymer zeigen im sowohl hinsichtlich der Hysterese als auch dem Schuhcremetest und dem IKW-Test hervorragende Ergebnisse.

## Patentansprüche

1. Beschichtungen, die eine mittels des Tilting-Plate-Verfahrens gemessene Kontaktwinkelhysterese von Wasser von höchstens 20° besitzen und herstellbar sind aus einer Mischung umfassend mindestens zwei unterschiedliche, untereinander und mit der Oberfläche des zu beschichtenden Substrates vernetzbaren sternförmigen Präpolymeren und/oder sternförmigen Präpolymer-Nanoteilchen-Komplexen, wobei die sternförmigen Präpolymere und/oder sternförmigen Präpolymer-Nanoteilchen-Komplexe vor ihrer Vernetzung mindestens drei hydrophile Polymerarme besitzen, die für sich gesehen in Wasser löslich sind und die an allen oder einem Teil ihrer freien Enden Silyl-Endgruppen R¹ der folgenden allgemeinen Formel (I)
R¹ ist -CR^{a}₂-Si(OR^{b})ᵣ(R^{c})₃₋ᵣ (I)
tragen, wobei R^{a} für Wasserstoff oder eine lineare oder verzweigte Alkylgruppe mit 1 bis 6 Kohlenstoffatomen steht, OR^{b} für eine hydrolysierbare Gruppe steht, R^{c} für eine lineare oder verzweigte Alkylgruppe mit 1 bis 6 Kohlenstoffatomen steht und r für eine Zahl von 1 bis 3 steht,
wobei die Silyl-Endgruppen R¹ nicht über ein Polyisocyanat am Ende des Polymerarms angebunden sind,
und an den gegebenenfalls vorhandenen nicht Silyl-Endgruppen-tragenden Enden reaktive Gruppen tragen, welche gegenüber sich selbst, dem zu beschichtenden Substrat, optional in die Beschichtung eingebrachten Entities und/oder mit den Silyl- Endgruppen reaktiv sind, mit der Maßgabe, dass die Mischung: (a) mindestens ein sternförmiges Präpolymer, welches 3 bis 5 hydrophile Polymerarme besitzt und (b) mindestens ein sternförmiges Präpolymer und/oder einen sternförmigen Präpolymer-Nanoteilchen-Komplex mit mindestens 6 hydrophilen Polymerarmen, enthält.

2. Beschichtungen nach Anspruch 1, wobei das sternförmige Präpolymer und/oder der sternförmige Präpolymer-Nanoteilchen-Komplex mehrere an eine Zentraleinheit gebundene Polymerketten aufweist, wobei die Zentraleinheit im Falle des sternförmigen Präpolymers eine niedermolekulare organochemische Zentraleinheit und im Falle des sternförmigen Präpolymer-Nanoteilchen-Komplexes ein anorganisches oxidisches Nanoteilchen darstellt, und wobei das sternförmige Präpolymer und/oder der sternförmige Präpolymer-Nanoteilchen-Komplex die nachfolgende allgemeine Formel (II) besitzt:
(R²-B-A-X)ₙ-Z-(X-A-B-R¹)ₘ (II)
worin
Z für die Zentraleinheit steht, wobei diese im Fall der sternförmigen Präpolymere die Armanzahl der mehrarmigen Präpolymere festlegt,
A für einen hydrophilen, für sich gesehen in Wasser löslichen Polymerarm steht,
B und X unabhängig voneinander für eine chemische Bindung oder einen zweiwertigen, niedermolekularen organischen Rest mit vorzugsweise 1 bis 50 Kohlenstoffatomen steht,
R² ungleich R¹ ist und für eine mit R¹, dem Substrat, den optional in die Beschichtung eingebrachten Entities und/oder mit sich selbst vernetzbare Gruppe steht, und
m und n jeweils ganze Zahlen sind, wobei m ≥ 1 und n ≥ 0 und m+n einen Wert von 3 bis 100 besitzt und mit der Armanzahl von Z übereinstimmt, und die m X-A-B-R¹-Gruppen sowie die n X-A-B-R²-Gruppen voneinander unabhängig verschiedene Bedeutung besitzen können.

3. Beschichtungen nach Anspruch 1 oder 2, wobei der Rest OR^{b} ein Alkoxy-Rest, vorzugsweise ein Methoxy- oder Ethoxy-Rest, und r = 1, 2, oder 3 ist.

4. Beschichtungen nach einem oder mehreren der Ansprüche 1 bis 3, wobei der Rest R² gewählt ist aus der Gruppe bestehend aus Isocyanat-Resten, (Meth)acrylat-Resten, Oxiran-Resten, alkoholischen OH-Gruppen, primären und sekundären Aminogruppen, Thiolgruppen und Silangruppen.

5. Beschichtungen nach einem oder mehreren der Ansprüche 1 bis 4, wobei die Polymerarme A ausgewählt sind aus der Gruppe bestehend aus Poly-C₂-C₄- alkylenoxiden, vorzugsweise aus Polyethylenoxid oder Ethylenoxid/Propylenoxid-Copolymeren, Polyoxazolidonon, Polyvinylalkoholen, Homo- und Copolymeren, die wenigstens 50 Gew.-% N-Vinylpyrrolidon einpolymerisiert enthalten, Homo- und Copolymeren, die wenigstens 30 Gew.-% Acrylamid und/oder Methacrylamid einpolymerisiert enthalten, Homo- und Copolymeren, die wenigstens 30 Gew.-% Acrylsäure und/oder Methacrylsäure einpolymerisiert enthalten.

6. Beschichtungen nach einem oder mehreren der Ansprüche 1 bis 5, wobei die sternförmigen Präpolymere durch Umsetzung von Verbindungen der allgemeinen Formel Z-(X-A-Y)ₘ₊ₙ, worin Z, X, A, m und n wie in Anspruch 2 definiert sind und Y für OH oder NH₂ steht, mit gegenüber der Gruppe Y reaktiven Silanen erhalten werden und die Verbindungen der allgemeinen Formel Z-(X-A-Y)ₘ₊ₙ ein zahlenmittleres Molekulargewicht im Bereich von 200 bis 50000 g/mol aufweisen, wobei das zahlenmittlere Molekulargewicht der Verbindungen der allgemeinen Formel Z-(X-A-Y)ₘ₊ₙ vorzugsweise 2000 bis 20000 g/mol beträgt.

7. Beschichtung nach einem oder mehreren der Ansprüche 1 bis 6, wobei die Beschichtung zusätzlich ein oder mehrere Entities, gewählt aus der Gruppe bestehend aus biologisch aktiven Substanzen, Pigmenten, Farbstoffen, Füllstoffen, Kieselsäureeinheiten, Nanopartikeln, funktionellen Organosilanen, biologischen Zellen, Rezeptoren oder Rezeptor tragenden Molekülen oder Zellen physikalisch eingelagert und/oder an diese oder in dieser kovalent gebunden, enthält.

8. Verfahren zur Herstellung einer Beschichtung wie sie in Ansprüchen 1 bis 7 definiert ist, auf einem Substrat, wobei eine Lösung der Mischung aus (a) und (b) wie in einem der Ansprüche 1 bis 7 definiert auf das zu beschichtende Substrat aufgebracht wird und vorher, gleichzeitig oder anschließend eine zumindest teilweise Vernetzungsreaktion der Silyl-Endgruppen und der gegebenenfalls vorhandenen reaktiven Gruppen der nicht Silyl- Endgruppen-tragenden Enden untereinander und/oder mit dem Substrat erfolgt.

9. Mischung sternförmiger Präpolymere, die mehrere an eine niedermolekulare Zentraleinheit gebundene Polymerketten aufweisen, und welche die nachfolgende allgemeine Formel (II) besitzen:
(R²-B-A-X)ₙ-Z-(X-A-B-R¹)ₘ (II)
worin
Z für die niedermolekulare Zentraleinheit steht, welche die Armanzahl der sternförmigen Präpolymere festlegt,
A für einen hydrophilen, für sich gesehen in Wasser löslichen Polymerarm steht,
B und X unabhängig voneinander für eine chemische Bindung oder einen zweiwertigen, niedermolekularen organischen Rest mit vorzugsweise 1 bis 50, insbesondere 2 bis 20 C-Atomen steht,
R¹ für eine Silyl-Gruppe der folgenden allgemeinen Formel (I)
-CR^{a}₂-Si(OR^{b})ᵣ(R^{c})₃₋ᵣ (I)
steht, wobei R^{a} für Wasserstoff oder eine lineare oder verzweigte Alkylgruppe mit 1 bis 6 Kohlenstoffatomen steht, OR^{b} für eine hydrolysierbare Gruppe steht, R^{c} für eine lineare oder verzweigte Alkylgruppe mit 1 bis 6 Kohlenstoffatomen steht und r für eine Zahl von 1 bis 3 steht,
wobei die Silyl-Endgruppen R¹ nicht über ein Polyisocyanat am Ende des Polymerarms angebunden sind,
R² ungleich R¹ und OH ist und für eine mit R¹, Substraten, Entities und/oder mit sich selbst vernetzbare oder reaktive Gruppe steht, und
m und n jeweils ganze Zahlen sind, wobei m ≥ 1 und n ≥ 1 und m+n einen Wert von 4 bis 100, besitzt und mit der Armanzahl von Z übereinstimmt,
und die m X-A-B-R¹-Gruppen sowie die n X-A-B-R²-Gruppen voneinander unabhängig verschiedene Bedeutung besitzen können, mit der Maßgabe, dass die Mischung (a) mindestens ein sternförmiges Präpolymer enthält, welches 3 bis 5 hydrophile Polymerarme besitzt und (b) mindestens ein sternförmiges Präpolymer mit mindestens 6 hydrophilen Polymerarmen.

10. Verwendung von Mischungen sternförmiger Präpolymere oder deren Derivate und/oder der in den erfindungsgemäßen Beschichtungen eingesetzten Mischungen wie sie nach den Ansprüchen 1 bis 7 und 9 definiert sind in Anti-Soiling-Mitteln zur temporären oder permanenten Ausrüstung von Oberflächen.

11. Verwendung von Mischungen sternförmiger Präpolymere oder deren Derivate und/oder der in den erfindungsgemäßen Beschichtungen eingesetzten Mischungen wie sie nach den Ansprüchen 1 bis 7 und 9 definiert sind als Additive in Reinigungsmitteln und Waschmitteln für harte und weiche Oberflächen, vorzugsweise für textile Oberflächen, Faseroberflächen oder Haaroberflächen, Haarpflegemitteln, Textilbehandlungsmitteln, Wand-, Fassaden- und Fugenbehandlungsmitteln, Mitteln zur Behandlung von Fahrzeugen und Mitteln zur Innen- und Außenbeschichtung von Behältern, Bioreaktoren und Wärmeaustauschern.

12. Verwendung von Mischungen sternförmiger Präpolymere oder deren Derivate und/oder der in den erfindungsgemäßen Beschichtungen eingesetzten Mischungen wie sie nach den Ansprüchen 1 bis 7 und 9 definiert sind zur Herstellung von Mikroarrays und Mikrosensoren für analytische Zwecke oder zur Beschichtung von Mikrokanülen oder Kapillaren.

13. Verwendung von Mischungen sternförmiger Präpolymere oder deren Derivate und/oder der in den erfindungsgemäßen Beschichtungen eingesetzten Mischungen wie sie nach den Ansprüchen 1 bis 7 und 9 definiert sind zur Reibungsverringerung von Oberflächen, Verringerung der elektrostatischen Aufladung von Oberflächen oder Fixierung von Farbstoffen auf Oberflächen.

14. Verwendung von Mischungen sternförmiger Präpolymere oder deren Derivate und/oder der in den erfindungsgemäßen Beschichtungen eingesetzten Mischungen wie sie nach den Ansprüchen 1 bis 7 und 9 definiert sind zur Herstellung von Oberflächenbeschichtungen, die ein gezieltes Aufwachsen von Feststoffen auf der beschichteten Oberfläche ermöglichen.

15. Anti-Soiling-Mittel, Reinigungsmittel und Waschmittel für harte und weiche Oberflächen, Haarpflegemittel, Textilbehandlungsmittel, Wand-, Fassaden- und Fugenbehandlungsmittel, Mittel zur Behandlung von Fahrzeugen, Mittel zur Innen- und Außenbeschichtung von Behältern, Bioreaktoren und Wärmeaustauschern enthaltend Mischungen sternförmiger Präpolymere oder deren Derivate wie sie in Anspruch 9 definiert sind.

## Claims

1. Coatings having a contact angle hysteresis of water of no more than 20°, measured by the tilting plate method and producible from a mixture comprising at least two different stellate prepolymers and/or stellate prepolymer-nanoparticle complexes, self-crosslinking and crosslinking with the surface of the substrate to be coated, the stellate prepolymers and/or stellate prepolymer-nanoparticle complexes having at least three hydrophilic polymer arms before their crosslinking, each being separately water-soluble and having silyl end groups R¹ of the following general formula (I) on some or all of their free ends:
R¹ is -CR^{a}₂-Si(OR^{b})ᵣ(R^{c})₃₋ᵣ (I)
in which R^{a} stands for hydrogen or a linear or branched alkyl group with one to six carbon atoms, OR^{b} stands for a hydrolyzable group, R^{c} stands for a linear or branched alkyl group with one to six carbon atoms, and r stands for a number from 1 to 3,
whereby the silyl end groups R¹ are not bound at the end of the polymer arm via a polyisocyanate,
and having reactive groups on the non-silyl-end-group-carrying ends, which are optionally present, these reactive groups being self-reactive, with the substrate to be coated, entities optionally introduced into the coating and/or reactive with the silyl end groups, with the provision that the mixture contains: (a) at least one stellate prepolymer having 3 to 5 hydrophilic polymer arms and (b) at least one stellate prepolymer and/or a stellate prepolymer-nanoparticle complex having at least 6 hydrophilic polymer arms.

2. The coatings according to claim 1, wherein the stellate prepolymer and/or the stellate prepolymer-nanoparticle complex has a plurality of polymer chains bound to a central unit, the central unit in the case of the stellate prepolymer being a low-molecular organochemical central unit, and in the case of the stellate prepolymer-nanoparticle complex, being an inorganic oxidic nanoparticle, and the stellate prepolymer and/or stellate prepolymer-nanoparticle complex has the following general formula (II)
(R²-B-A-X)ₙ-Z-(X-A-B-R¹)ₘ (II)
in which
Z stands for the central unit, which, in the case of stellate prepolymers, determines the number of arms of the multiarmed prepolymer,
A stands for a hydrophilic, separately water-soluble polymer arm,
B and X, independently of one another, stand for a chemical bond or a divalent, low-molecular, organic radical, preferably having 1 to 50 carbon atoms,
R² is not equal to R¹ and stands for a group that is self-crosslinkable and/or is crosslinkable with R¹, the substrate, the entities optionally introduced into the coating, and
m and n are each integers, where m ≥ 1 and n ≥ 0, and m + n have a value from 3 to 100 and corresponds to the number of arms of Z, and the m X-A-B-R¹ groups and the n X-A-B-R² groups may have different meanings independently of one another.

3. The coatings according to claim 1 or 2, wherein the OR^{b} radical is an alkoxy radical, preferably a methoxy or ethoxy radical, and r = 1, 2 or 3.

4. The coatings according to any one or more of claims 1 to 3, wherein the R² radical is selected from the group consisting of isocyanate radicals, (meth)acrylate radicals, oxirane radicals, alcoholic OH groups, primary and secondary amino groups, thiol groups and silane groups.

5. The coatings according to any one or more of claims 1 to 4, wherein the polymer arms A are selected from the group consisting of poly-C₂-C₄-alkylene oxides, preferably consisting of polyethylene oxide or ethylene oxide/propylene oxide copolymers, polyoxazolidones, polyvinyl alcohols, homopolymers and copolymers containing at least 50 wt% N-vinylpyrrolidone polymerized into them, homopolymers and copolymers containing at least 30 wt% acrylamide and/or methacrylamide polymerized into them, homopolymers and copolymers containing at least 30 wt% acrylic acid and/or methacrylic acid polymerized into them.

6. The coatings according to any one or more of claims 1 to 5, wherein the stellate prepolymers are obtained by reacting compounds of general formula Z-(X-A- Y)ₘ₊ₙ, in which Z, X, A, m and n are defined as in claim 2, and Y stands for OH or NH₂, with silanes that are reactive with the Y group, and the compounds of general formula Z-(X-A-Y)ₘ₊ₙ have a number-average molecular weight in the range of 200 to 50,000 g/mol, the number-average molecular weight of the compounds of general formula Z-(X-A-Y)ₘ₊ₙ preferably being 2,000 to 20,000 g/mol.

7. The coating according to any one or more of claims 1 to 6, wherein the coating contains one or more entities selected from the group consisting of biologically active substances, pigments, dyes, fillers, silicic acid units, nanoparticles, functional organosilanes, biological cells, receptors or receptor-bearing molecules or cells, which are physically incorporated into the coating and/or covalently bonded onto or into it.

8. A method for producing a coating as defined in claims 1 to 7, on a substrate, wherein a solution of the mixture of (a) and (b) as defined in any one of claims 1 to 7 is applied to the substrate to be coated, and before, simultaneously or subsequently, an at least partial crosslinking reaction of the silyl end groups and the reactive groups optionally present on the non-silyl-end-group-carrying ends with one another and/or with the substrate is/are performed.

9. A mixture of stellate prepolymers having a plurality of polymer chains bound to a low-molecular central unit and having the following general formula (II):
(R²-B-A-X)ₙ-Z-(X-A-B-R¹)ₘ (II)
in which
Z stands for the low-molecular central unit, which defines the number of arms of the stellate prepolymer,
A stands for a hydrophilic, separately water-soluble polymer arm,
B and X, independently of one another, stand for a chemical bond or a divalent, low-molecular, organic radical, preferably having 1 to 50 C atoms, in particular 2 to 20 C atoms,
R¹ stands for a silyl group of the following general formula (I)
-CR^{a}₂-Si(OR^{b})ᵣ(R^{C})₃₋ᵣ (I)
in which R^{a} stands for hydrogen or a linear or branched alkyl group with one to six carbon atoms, OR^{b} stands for a hydrolyzable group, R^{c} stands for a linear or branched alkyl group with 1 to 6 carbon atoms,
and r stands for a number from 1 to 3,
in which the silyl end groups R¹ are not bound to the end of the polymer arm via a polyisocyanate,
R² is not equal to R¹ and is OH and stands for a group crosslinkable with R¹, substrates, entities and/or self-crosslinkable or for a reactive group, and
m and n are each integers, where m ≥ 1 and n ≥ 1, and m + n have a value from 4 to 100 and corresponds to the number of arms of Z,
and the m X-A-B-R¹ groups and the n X-A-B-R² groups may have different meanings independently of one another, with the provision that the mixture contains (a) at least one stellate prepolymer having 3 to 5 hydrophilic polymer arms and (b) at least one stellate prepolymer having at least six hydrophilic polymer arms.

10. Use of mixtures of stellate prepolymers or their derivatives and/or mixtures used in the coatings according to the invention, as defined according to claims 1 to 7 and 9, in antisoiling agents for temporary or permanent finishing of surfaces.

11. Use of mixtures of stellate prepolymers or their derivatives and/or the mixtures used in the coatings according to the invention, as defined according to claims 1 to 7 and 9, as additives in cleaning agents and washing agents for hard and soft surfaces, preferably for textile surfaces, fiber surfaces or hair surfaces, hair care agents, textile treatment agents, treatment agents for walls, facades and joints, agents for treatment of vehicles and agents for interior and exterior coating of containers, bioreactors and heat exchangers.

12. Use of mixtures of stellate prepolymers or their derivatives and/or the mixtures used in the coatings according to the invention, as defined according to claims 1 to 7 and 9, for production of microarrays and microsensors for analytical purposes or for coating of microcannulas or capillaries.

13. Use of mixtures of stellate prepolymers or their derivatives and/or mixtures used in the coatings according to the invention, as defined according to claims 1 to 7 and 9, for reducing the friction of surfaces, reducing the electrostatic charge of surfaces or fixation of dyes on surfaces.

14. Use of mixtures of stellate prepolymers or their derivatives and/or the mixtures used in the coatings according to the invention, as defined according to claims 1 to 7 and 9, for production of surface coatings, which allow targeted growth of solids on the coated surface.

15. Antisoiling agents, cleaning agents and washing agents for hard and soft surfaces, hair care agents, textile treatment agents, treatment agents for walls, facades and joints, agents for treatment of vehicles, agents for interior and exterior coating of containers, bioreactors and heat exchangers containing mixtures of stellate prepolymers or their derivatives, as defined in claim 9.

## Revendications

1. Enductions qui possèdent une hystérésis maximale de 20° de l'angle de contact, mesurée au moyen du procédé dit « de la plaque basculante » et que l'on peut préparer à partir d'un mélange comprenant au moins deux prépolymères en étoile et/ou des complexes de nanoparticules prépolymères en étoile qui peuvent être soumis à une réticulation avec la surface du substrat à enduire, les prépolymères en étoile et/ou les complexes de nanoparticules prépolymères en étoile possédant, avant leur réticulation, au moins trois bras polymères hydrophiles qui, en soi, sont solubles dans l'eau et qui portent, à toutes leurs extrémités libres ou à une partie de ces dernières, des groupes silyle terminaux R¹ répondant à la formule générale suivante (1)
R¹ représente un groupe
-CR^{a}₂-Si(OR^{b})ᵣ(R^{c})₃₋ᵣ (I)
dans lequel R^{a} représente un atome d'hydrogène ou un groupe alkyle linéaire ou ramifié contenant de 1 à 6 atomes de carbone, OR^{b} représente un groupe hydrolysable, R^{c} représente un groupe alkyle linéaire ou ramifié contenant de 1 à 6 atomes de carbone, et r représente un nombre de 1 à 3 ;
les groupes silyle terminaux R¹ n'étant pas liés via un polyisocyanate à l'extrémité du bras polymère ;
et qui portent, aux extrémités éventuellement présentes qui ne portent pas de groupes silyle terminaux, des groupes réactifs qui sont à même de réagir avec eux-mêmes, avec le substrat à enduire, avec des entités incorporées de manière facultative dans l'enduction et/ou avec les groupes silyle terminaux, avec cette mesure que le mélange contient : (a) au moins un prépolymère en étoile qui possède de 3 à 5 bras polymères hydrophiles et (b) au moins un prépolymère en étoile et/ou un complexe
de nanoparticules prépolymères en étoile comprenant au moins 6 bras polymères hydrophiles.

2. Enductions selon la revendication 1, dans lesquelles le prépolymère en étoile et/ou le complexe de nanoparticules prépolymères en étoile présentent plusieurs chaînes polymères liées à une unité centrale, l'unité centrale représentant, dans le cas du prépolymère en étoile, une unité centrale organochimique à bas poids moléculaire et dans le cas du complexe de nanoparticules prépolymères en étoile, une nanoparticule oxydique inorganique, et dans lesquelles le prépolymère en étoile et/ou le complexe de nanoparticules prépolymères en étoile répondent à la formule générale suivante (II) :
(R²-B-A-X)ₙ-Z-(X-A-B-R¹)ₘ (II)
dans laquelle
Z représente l'unité centrale, celle-ci déterminant, dans le cas des prépolymères en étoile, le nombre de bras des prépolymères à plusieurs bras ;
A représente un bras polymère hydrophile en soi soluble dans l'eau ;
B et X représentent, indépendamment l'un de l'autre, une liaison chimique ou bien un résidu organique bivalent à bas poids moléculaire comprenant de préférence de 1 à 50 atomes de carbone ;
R² n'est pas égal à R¹ et représente un groupe réticulable avec R¹, avec le substrat, avec les entités incorporées de manière facultative dans l'enduction et/ou avec lui-même, et
m et n représentent respectivement des nombres entiers, m étant supérieur ou égal à 1 et n étant supérieur ou égal à 0 et m+n possédant une valeur de 3 à 100 et correspondant au nombre de bras de Z, et
les m groupes X-A-B-R¹ ainsi que les m groupes X-A-B-R² peuvent avoir des significations différentes, indépendamment les uns des autres.

3. Enductions selon la revendication 1 ou 2, dans lesquelles le résidu OR^{b} représente un résidu alcoxy, de préférence un résidu méthoxy ou éthoxy, et r est égal à 1,2 ou 3.

4. Enductions selon une ou plusieurs des revendications 1 à 3, dans lesquelles le résidu R² est choisi parmi le groupe constitué par des résidus isocyanate, des résidus (méth)acrylate, des résidus oxirane, des groupes OH alcooliques, des groupes amino primaires et secondaires, des groupes thiol et des groupes silane.

5. Enductions selon une ou plusieurs des revendications 1 à 4, dans lesquelles les bras polymères A sont choisis parmi le groupe constitué par des oxydes de polyalkylènes en C₂-C₄, de préférence par des copolymères d'oxyde de polyéthylène ou d'oxyde d'éthylène/oxyde de propylène, des polyoxazolidones, des alcools polyvinyliques, des homopolymères et des copolymères qui contiennent au moins 50 % en poids de N-vinylpyrrolidone incorporée par polymérisation, des homopolymères et des copolymères qui contiennent au moins 30 % en poids d'acrylamide et/ou de méthacrylamide incorporés par polymérisation, des homopolymères et des copolymères qui contiennent au moins 30 % en poids d'acide acrylique et/ou d'acide méthacrylique incorporés par polymérisation.

6. Enductions selon une ou plusieurs des revendications 1 à 5, dans lesquelles on obtient les prépolymères en étoile par mise en réaction de composés répondant à la formule générale Z-(X-A-Y)ₘ₊ₙ, dans laquelle Z, X, A, m et n sont tels que définis à la revendication 2, et Y représente un groupe OH ou un groupe NH₂, avec des silanes aptes à réagir avec le groupe Y, et les composés répondant à la formule générale Z-(X-A-Y)ₘ₊ₙ présentent un poids moléculaire moyen en nombre dans la plage de 200 à 50.000 g/mol, le poids moléculaire moyen en nombre des composés
répondant à la formule générale Z-(X-A-Y)ₘ₊ₙ s'élevant de préférence de 2.000 à 20.000 g/mol.

7. Enduction selon une ou plusieurs des revendications 1 à 6, dans laquelle on incorpore par voie physique dans l'enduction en outre une ou plusieurs entités, choisies parmi le groupe constitué par des substances biologiquement actives, des pigments, des colorants, des matières de charge, des unités d'acide silicique, des nanoparticules, des organosilanes fonctionnels, des cellules biologiques, des récepteurs ou bien des molécules ou des cellules portant des récepteurs et/ou on lie lesdites entités de manière covalente à ladite enduction ou dans ladite enduction.

8. Procédé pour la préparation d'une enduction telle qu'elle est définie aux revendications 1 à 7, sur un substrat, dans lequel on applique sur le substrat à enduire une solution du mélange de (a) et (b) comme défini dans l'une quelconque des revendications 1 à 7, et auparavant, de manière simultanée ou directement après on procède à une réaction de réticulation au moins partielle des groupes silyle terminaux et des groupes réactifs éventuellement présents des extrémités qui ne portent pas des groupes silyle terminaux et/ou avec le substrat.

9. Mélange de prépolymères en étoile qui présentent plusieurs chaînes polymères liées à une unité centrale à bas poids moléculaire et qui répondent à la formule générale suivante (II)
(R²-B-A-X)ₙ-Z-(X-A-B-R¹)ₘ (II)
dans laquelle
Z représente l'unité centrale à bas poids moléculaire qui détermine le nombre de bras des prépolymères en étoile ;
A représente un bras polymère hydrophile en soi soluble dans l'eau ;
B et X représentent, indépendamment l'un de l'autre, une liaison chimique ou bien un résidu organique bivalent à bas poids moléculaire comprenant de préférence de 1 à 50, en particulier de 2 à 20 atomes de carbone ;
R¹ représente un groupe silyle répondant à la formule générale suivante (I)
-CR^{a}₂-Si(OR^{b})ᵣ(R^{c})₃₋ᵣ (I)
dans laquelle R^{a} représente un atome d'hydrogène ou un groupe alkyle linéaire ou ramifié contenant de 1 à 6 atomes de carbone, OR^{b} représente un groupe hydrolysable, R^{c} représente un groupe alkyle linéaire ou ramifié contenant de 1 à 6 atomes de carbone, et r représente un nombre de 1 à 3 ;
les groupes silyle terminaux R¹ n'étant pas liés via un polyisocyanate à l'extrémité du bras polymère ;
R² n'est pas égal à R¹ et représente un groupe OH et représente un groupe réticulable ou apte à réagir avec R¹, avec des substrats, avec des entités et/ou avec lui-même, et
m et n représentent respectivement des nombres entiers, m étant supérieur ou égal à 1 et n étant supérieur ou égal à 1 et m+n possédant une valeur de 4 à 100, et correspondant au nombre de bras de Z, et
les m groupes X-A-B-R¹ ainsi que les m groupes X-A-B-R² peuvent posséder des significations différentes, indépendamment les uns des autres, avec cette mesure que le mélange contient : (a) au moins un prépolymère en étoile qui possède de 3 à 5 bras polymères hydrophiles et (b) au moins un prépolymère en étoile comprenant au moins 6 bras polymères hydrophiles.

10. Utilisation de mélanges de prépolymères en étoile ou de leurs dérivés et/ou des mélanges mis en oeuvre dans les enductions selon l'invention, tels qu'ils sont définis conformément aux revendications 1 à 7 et 9, dans des agents antisalissure pour le traitement temporaire ou permanent de surfaces.

11. Utilisation de mélanges de prépolymères en étoile ou de leurs dérivés et/ou des mélanges mis en oeuvre dans les enductions selon l'invention, tels qu'ils sont définis conformément aux revendications 1 à 7 et 9, comme additifs dans des agents de nettoyage et des agents de lavage pour des surfaces dures et tendres, de préférence pour des surfaces textiles, pour des surfaces fibreuses ou pour des surfaces capillaires, dans des agents de soins capillaires, dans des agents de traitement des textiles, dans des agents de traitement des murs, des façades et des joints, dans des agents pour l'entretien de véhicules, et dans des agents pour l'enduction interne et externe de récipients, de bioréacteurs et d'échangeurs de chaleur.

12. Utilisation de mélanges de prépolymères en étoile ou de leurs dérivés et/ou des mélanges mis en oeuvre dans les enductions selon l'invention, tels qu'ils sont définis conformément aux revendications 1 à 7 et 9, pour la fabrication de microarrays à ADN et de microcapteurs pour des objets analytiques ou bien pour l'enduction de microcanules ou de capillaires.

13. Utilisation de mélanges de prépolymères en étoile ou de leurs dérivés et/ou des mélanges mis en oeuvre dans les enductions selon l'invention, tels qu'ils sont définis conformément aux revendications 1 à 7 et 9, pour réduire la friction de surface, pour réduire la charge électrostatique de surfaces ou bien pour fixer des colorants sur des surfaces.

14. Utilisation de mélanges de prépolymères en étoile ou de leurs dérivés et/ou des mélanges mis en oeuvre dans les enductions selon l'invention, tels qu'ils sont définis conformément aux revendications 1 à 7 et 9, pour la préparation d'enductions de surfaces qui permettent une croissance ciblée de substances solides sur la surface enduite.

15. Agent antisalissure, agent de nettoyage et agent de lavage pour des surfaces dures et tendres, pour des agents de soins capillaires, pour des agents de traitement des textiles, pour des agents de traitement des murs, des façades et des joints, pour des agents destinés à l'entretien de véhicules, pour des agents destinés à l'enduction interne et externe de récipients de bioréacteurs et d'échangeurs de chaleur, contenant des mélanges de prépolymères en étoile ou de leurs dérivés, tels qu'ils sont définis à la revendication 9.
